# EUROPEAN PATENT SPECIFICATION

(11) **EP 4 164 515 B1**
(45) Date of publication and mention of the grant of the patent: **15.10.2025**
(21) Application number: 22729809.8
(22) Date of filing: 25.05.2022
(51) Int. Cl.: A61B 17/072, A61B 17/00, A61B 90/00

(54) **STAPLING INSTRUMENT COMPRISING A FIRING LOCKOUT**
KLAMMERINSTRUMENT MIT EINER SCHUSSSPERRE
INSTRUMENT D'AGRAFAGE COMPRENANT UN VERROUILLAGE DE TIR

(30) Priority: 28.05.2021 US 202163194621 P; 18.08.2021 US 202163234396 P; 25.04.2022 US 202217728076
(43) Date of publication of application: 19.04.2023
(73) Proprietor: Cilag GmbH International, 6300 Zug (CH)
(72) Inventor: HUANG, Zhifan F., Cincinnati, Ohio 45242 (US); LEIMBACH, Richard L., Cincinnati, Ohio 45242 (US); HUEIL, Geoffrey C., Cincinnati, Ohio 45242 (US); ROSS, Nicholas J., Cincinnati, Ohio 45242 (US); SPINNEWEBER, Brian K., Cincinnati, Ohio 45242 (US); RUOFF, Joshua R., Cincinnati, Ohio 45242 (US); JOHNSON, Robbin B., Cincinnati, Ohio 45242 (US); BABER, Daniel L., Cincinnati, Ohio 45242 (US); MESNIL, Pierre R., Cincinnati, Ohio 45242 (US)
(74) Representative: Carpmaels & Ransford LLP
(86) International application number: PCT/IB2022/054905
(87) International publication number: WO 2022/249099

(56) References cited:
- WO-A1-2018/116013
- WO-A1-2019/186431

## Description

### BACKGROUND

The present invention relates to surgical instruments and, in various arrangements, to surgical stapling and cutting instruments and staple cartridges for use therewith that are designed to staple and cut tissue.

WO 2018/116013 A discloses a surgical stapling assembly comprising interchangeable surgical tool assemblies which include an end effector including a first jaw, a second jaw, and a sled configured to translate relative to the first jaw and the second jaw. The interchangeable surgical tool assemblies also include a firing member with an I-beam structure, comprising a first flange configured to engage the first jaw and a second flange configured to engage the second jaw. The assemblies further include a pusher plate and a firing bar selectively coupled to the pusher plate, wherein the firing bar is configured to move through a plurality of successive firing strokes including a first distal firing stroke, a first proximal firing stroke, a second distal firing stroke, and a second proximal firing stroke. A disclosed end effector includes a lockout arrangement which includes a lock having first, second and third legs and a lockout spring which is configured to act on the lock. WO 2019/186431 A1 discloses a surgical stapling assembly comprising a stapling instrument configured to receive a staple cartridge therein. The stapling instrument comprises a first jaw, a second jaw movable relative to the first jaw, a firing member configured to push a sled to deploy staples, and a lockout configured to prevent the firing member from advancing distally when the staple cartridge is not properly seated within the stapling instrument and when the sled of the staple cartridge is not in an unfired position, wherein a lockout key of the staple cartridge defeats a first stage of the lockout when the staple cartridge is fully seated in the stapling instrument, and wherein the sled defeats a second stage of the lockout when the sled is in the unfired position and the staple cartridge is fully seated in the stapling instrument.

### SUMMARY OF THE INVENTION

The present invention is defined in independent claim 1. Optional features are recited in the dependent claims. Aspects, embodiments, examples and methods of the present disclosure which are not claimed per se, are provided for illustrative purposes and are considered useful for giving context to the invention.

### BRIEF DESCRIPTION OF THE DRAWINGS

Various features of the embodiments described herein, together with advantages thereof, may be understood in accordance with the following description taken in conjunction with the accompanying drawings as follows:
FIG. 1 is a perspective view of a surgical instrument in accordance with at least one embodiment;
FIG. 1B is a left side elevation view of the surgical instrument of FIG. 1;
FIG. 1C is a right side elevation view of the surgical instrument of FIG. 1;
FIG. 1D is a front elevation view of the surgical instrument of FIG. 1;
FIG. 1E is a back elevation view of the surgical instrument of FIG. 1;
FIG. 1F is a plan view of the surgical instrument of FIG. 1;
FIG. 1G is a bottom view of the surgical instrument of FIG. 1;
FIG. 2 is a partial perspective view of the surgical instrument of FIG. 1;
FIG. 3 is a partial perspective view of a shaft of the surgical instrument of FIG. 1;
FIG. 4 is a perspective view of a nozzle of the shaft of FIG. 3;
FIG. 5 is an elevational view of an orientation switch of the surgical instrument of FIG. 1;
FIG. 6 is an elevational view of a surgical instrument including a handle and a shaft in accordance with at least one embodiment;
FIG. 7 is a partial elevational view of the surgical instrument of FIG. 6 illustrated with some components removed;
FIG. 8 is a perspective view of a frame of the handle of FIG. 6 connected to a frame of the shaft of FIG. 6;
FIG. 9 is an exploded view of the handle frame and the shaft frame of FIG. 8;
FIG. 10 is a perspective view of the handle of FIG. 6;
FIG. 11 is a partial perspective view of the handle of FIG. 6 illustrated with some components removed;
FIG. 12 is a partial cross-sectional view of a switch of the handle of FIG. 6;
FIG. 13 is an elevational view of the handle of FIG. 6 illustrated with some components removed illustrating a closure actuator of the handle in a partially-closed position;
FIG. 14 is a partial detail view of the closure system of the handle of FIG. 6 illustrated in a partially-closed configuration;
FIG. 15 is a partial detail view of the closure system of the handle of FIG. 6 illustrated in a fully-closed configuration;
FIG. 16 is a partial perspective view of the handle of the surgical instrument of FIG. 6 illustrated with some components removed;
FIG. 17 is a partial perspective view of the closure system of the surgical instrument of FIG. 6;
FIG. 18 is a partial perspective view of a closure system in accordance with at least embodiment;
FIG. 19A depicts a spring of the closure system of FIG. 17;
FIG. 19B depicts a spring of a closure system in accordance with at least one embodiment;
FIG. 19C depicts a spring system of the closure system of FIG. 18;
FIG. 20 is a graph depicting the force generated by the spring system of FIG. 19C;
FIG. 21 is a partial plan view of a jaw of the end effector of the surgical instrument of FIG. 6 including a staple firing lockout;
FIG. 22 is a partial elevational view of a staple firing system and the staple firing lockout of FIG. 21;
FIG. 23 is a perspective view of a surgical instrument comprising a handle, a shaft extending from the handle, and an end effector rotatably connected to the shaft about an articulation joint in accordance with at least one embodiment;
FIG. 24 is an elevational view of the surgical instrument of FIG. 23;
FIG. 25 is a top view of the surgical instrument of FIG. 23;
FIG. 26 is an exploded view of the shaft of the surgical instrument of FIG. 23;
FIG. 27 is an exploded view of the articulation joint of the surgical instrument of FIG. 23;
FIG. 28 is an exploded view of the handle of the surgical instrument of FIG. 23;
FIG. 29 is a partial cross-sectional elevational view of the surgical instrument of FIG. 23 illustrating the end effector in an open configuration;
FIG. 30 is a partial cross-sectional elevational view of the surgical instrument of FIG. 23 illustrated with some components removed;
FIG. 31 is a perspective view of a firing member of the surgical instrument of FIG. 23;
FIG. 32 is a perspective view of an end effector of a surgical instrument in accordance with at least one embodiment illustrated without a staple cartridge seated in a cartridge jaw of the end effector;
FIG. 33 is a partial cross-sectional view of the end effector of FIG. 32 illustrated in an open configuration with a staple cartridge seated in the cartridge jaw;
FIG. 34 is a partial cross-sectional view of the end effector of FIG. 32 illustrated in a clamped configuration;

Corresponding reference characters indicate corresponding parts throughout the several views. The exemplifications set out herein illustrate various embodiments.

### DETAILED DESCRIPTION

Numerous specific details are set forth to provide a thorough understanding of the overall structure, function, manufacture, and use of the embodiments as described in the specification and illustrated in the accompanying drawings. Well-known operations, components, and elements have not been described in detail so as not to obscure the embodiments described in the specification. The reader will understand that the embodiments described and illustrated herein are non-limiting examples, and thus it can be appreciated that the specific structural and functional details disclosed herein may be representative and illustrative. Variations and changes thereto may be made without departing from the scope of the claims.

The terms "comprise" (and any form of comprise, such as "comprises" and "comprising"), "have" (and any form of have, such as "has" and "having"), "include" (and any form of include, such as "includes" and "including") and "contain" (and any form of contain, such as "contains" and "containing") are open-ended linking verbs. As a result, a surgical system, device, or apparatus that "comprises," "has," "includes" or "contains" one or more elements possesses those one or more elements, but is not limited to possessing only those one or more elements. Likewise, an element of a system, device, or apparatus that "comprises," "has," "includes" or "contains" one or more features possesses those one or more features, but is not limited to possessing only those one or more features.

The terms "proximal" and "distal" are used herein with reference to a clinician manipulating the handle portion of the surgical instrument. The term "proximal" refers to the portion closest to the clinician and the term "distal" refers to the portion located away from the clinician. It will be further appreciated that, for convenience and clarity, spatial terms such as "vertical", "horizontal", "up", and "down" may be used herein with respect to the drawings. However, surgical instruments are used in many orientations and positions, and these terms are not intended to be limiting and/or absolute.

Various exemplary devices and methods are provided for performing laparoscopic and minimally invasive surgical procedures. However, the reader will readily appreciate that the various methods and devices disclosed herein can be used in numerous surgical procedures and applications including, for example, in connection with open surgical procedures. As the present Detailed Description proceeds, the reader will further appreciate that the various instruments disclosed herein can be inserted into a body in any way, such as through a natural orifice, through an incision or puncture hole formed in tissue, etc. The working portions or end effector portions of the instruments can be inserted directly into a patient's body or can be inserted through an access device that has a working channel through which the end effector and elongate shaft of a surgical instrument can be advanced.

A surgical stapling system can comprise a shaft and an end effector extending from the shaft. The end effector comprises a first jaw and a second jaw. The first jaw comprises a staple cartridge. The staple cartridge is insertable into and removable from the first jaw; however, other embodiments are envisioned in which a staple cartridge is not removable from, or at least readily replaceable from, the first jaw. The second jaw comprises an anvil configured to deform staples ejected from the staple cartridge. The second jaw is pivotable relative to the first jaw about a closure axis; however, other embodiments are envisioned in which the first jaw is pivotable relative to the second jaw. The surgical stapling system further comprises an articulation joint configured to permit the end effector to be rotated, or articulated, relative to the shaft. The end effector is rotatable about an articulation axis extending through the articulation joint. Other embodiments are envisioned which do not include an articulation joint.

The staple cartridge comprises a cartridge body. The cartridge body includes a proximal end, a distal end, and a deck extending between the proximal end and the distal end. In use, the staple cartridge is positioned on a first side of the tissue to be stapled and the anvil is positioned on a second side of the tissue. The anvil is moved toward the staple cartridge to compress and clamp the tissue against the deck. Thereafter, staples removably stored in the cartridge body can be deployed into the tissue. The cartridge body includes staple cavities defined therein wherein staples are removably stored in the staple cavities. The staple cavities are arranged in six longitudinal rows. Three rows of staple cavities are positioned on a first side of a longitudinal slot and three rows of staple cavities are positioned on a second side of the longitudinal slot. Other arrangements of staple cavities and staples may be possible.

The staples are supported by staple drivers in the cartridge body. The drivers are movable between a first, or unfired position, and a second, or fired, position to eject the staples from the staple cavities. The drivers are retained in the cartridge body by a retainer which extends around the bottom of the cartridge body and includes resilient members configured to grip the cartridge body and hold the retainer to the cartridge body. The drivers are movable between their unfired positions and their fired positions by a sled. The sled is movable between a proximal position adjacent the proximal end and a distal position adjacent the distal end. The sled comprises a plurality of ramped surfaces configured to slide under the drivers and lift the drivers, and the staples supported thereon, toward the anvil.

Further to the above, the sled is moved distally by a firing member. The firing member is configured to contact the sled and push the sled toward the distal end. The longitudinal slot defined in the cartridge body is configured to receive the firing member. The anvil also includes a slot configured to receive the firing member. The firing member further comprises a first cam which engages the first jaw and a second cam which engages the second jaw. As the firing member is advanced distally, the first cam and the second cam can control the distance, or tissue gap, between the deck of the staple cartridge and the anvil. The firing member also comprises a knife configured to incise the tissue captured intermediate the staple cartridge and the anvil. It is desirable for the knife to be positioned at least partially proximal to the ramped surfaces such that the staples are ejected ahead of the knife.

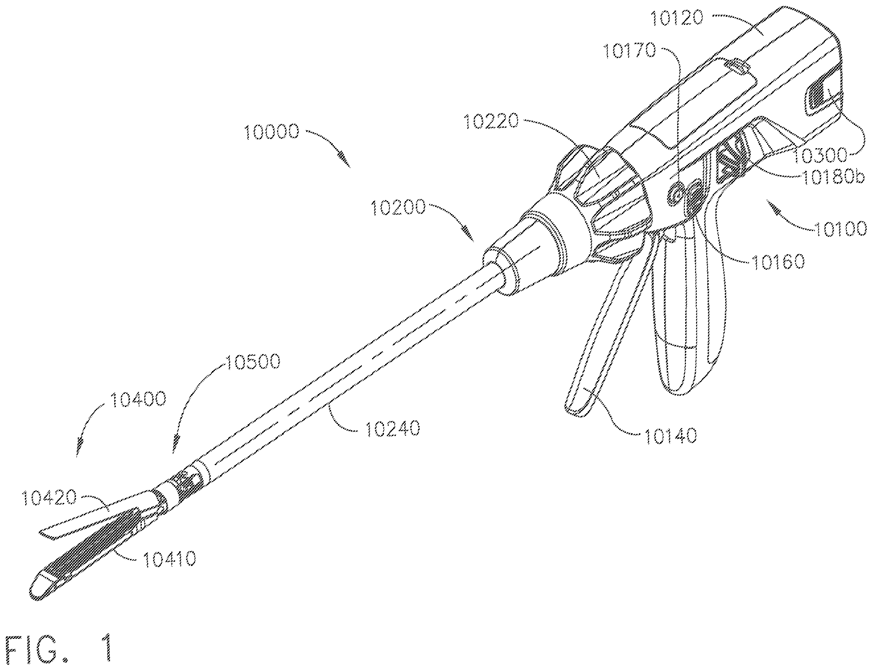

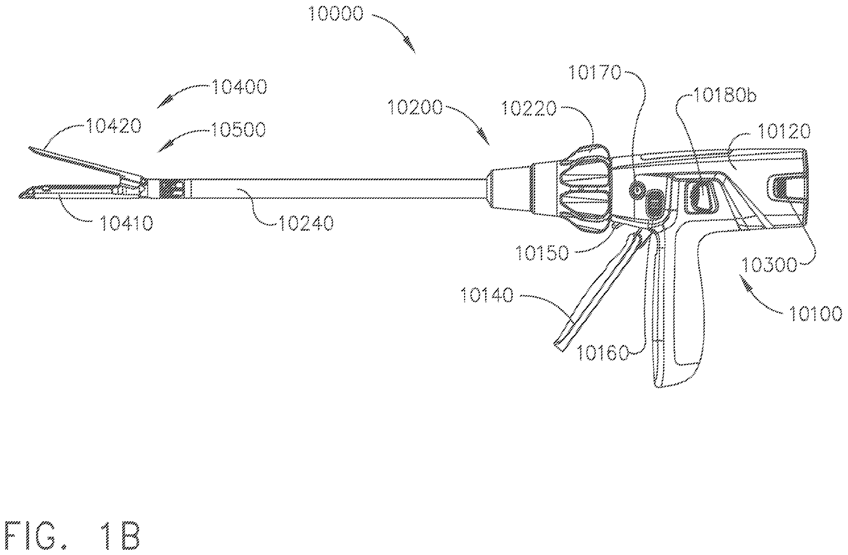

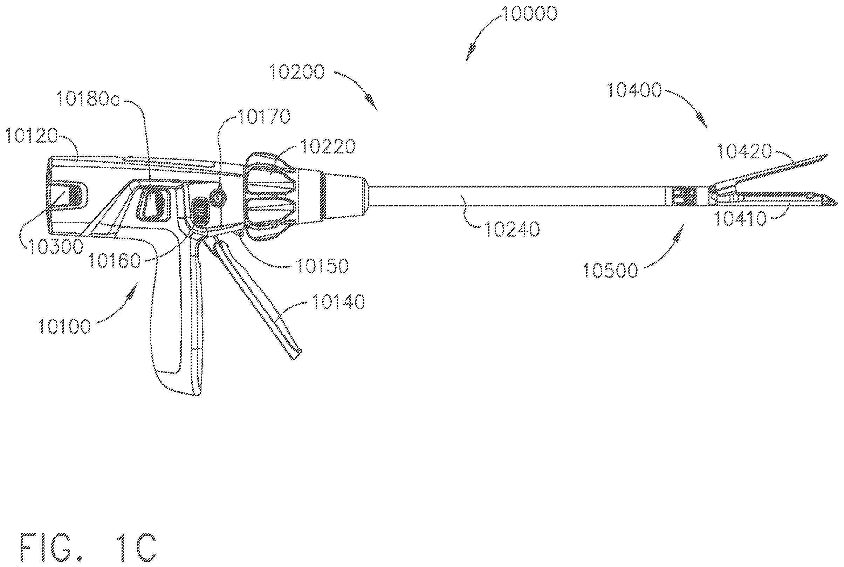

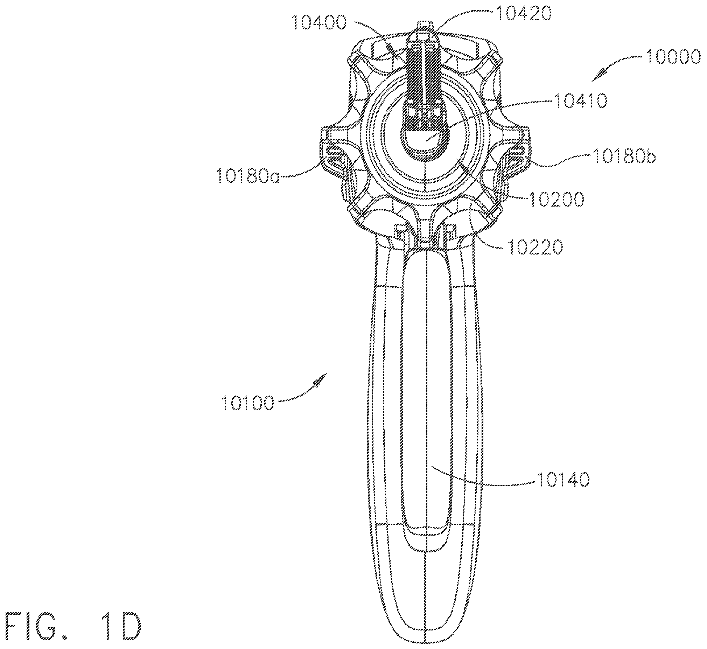

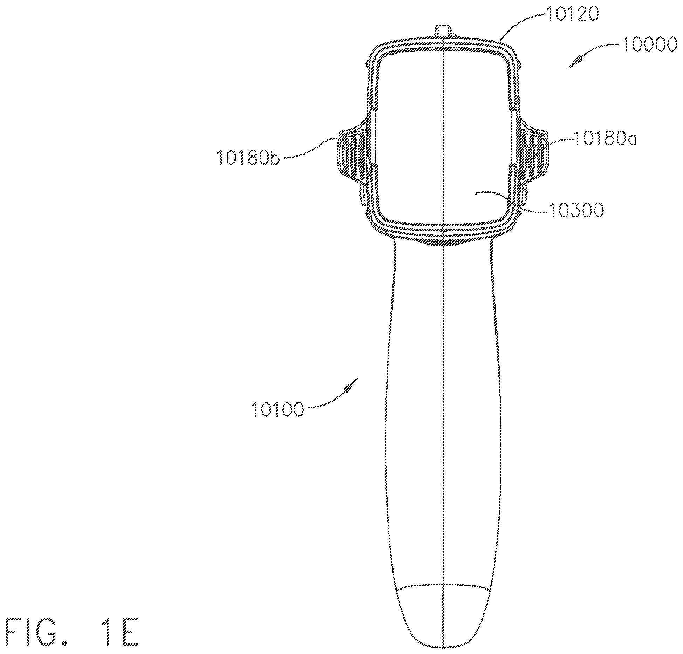

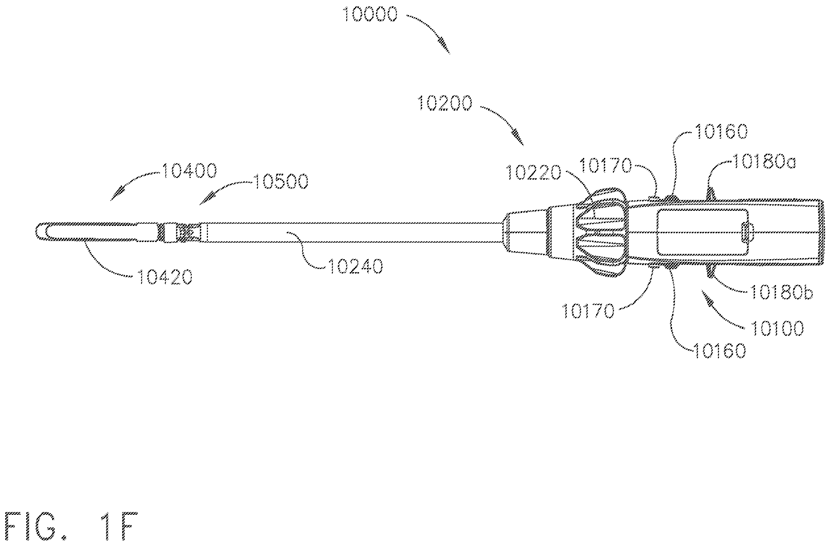

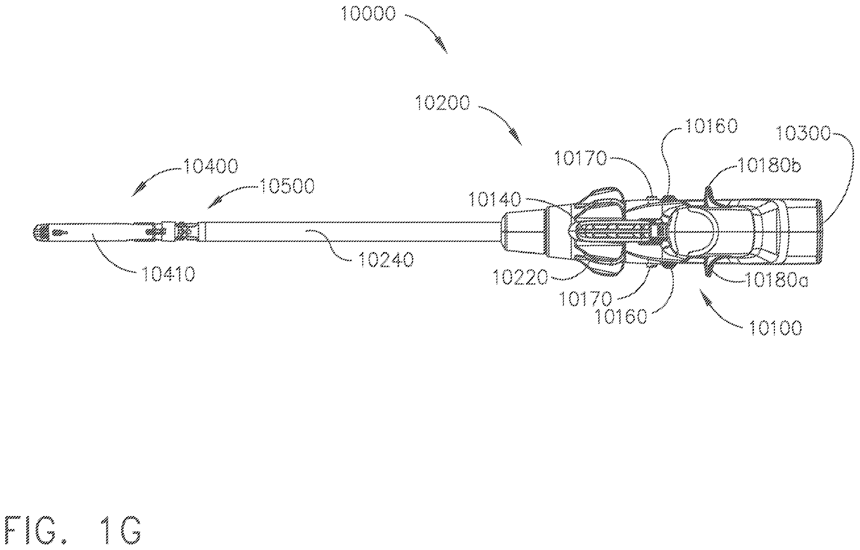

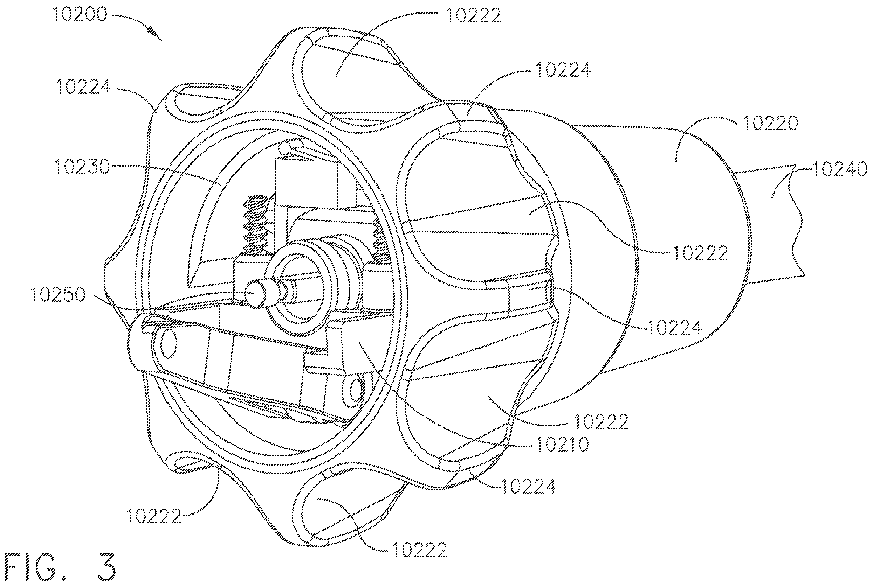

A surgical instrument 10000 is illustrated in FIG. 1. The surgical instrument 10000 comprises a handle 10100, a shaft 10200 extending from the handle 10100, and an end effector 10400. The end effector 10400 comprises a first jaw 10410 configured to receive a staple cartridge and a second jaw 10420 movable relative to the first jaw 10410. The second jaw 10420 comprises an anvil including staple forming pockets defined therein. The surgical instrument 10000 further comprises a closure actuator 10140 configured to drive a closure system of the surgical instrument 10000 and move the second jaw 10420 between an unclamped position and a clamped position. Referring to FIG. 3, the closure actuator 10140 is operably coupled with a closure tube 10240 that is advanced distally when the closure actuator 10140 is closed. In such instances, the closure tube 10240 contacts the second jaw and cams and/or pushes the second jaw 10420 downwardly into its clamped position. The second jaw 10420 is pivotably coupled to the first jaw about a pivot axis. That said, in alternative embodiments, the second jaw can translate and rotate as it is being moved into its clamped position. Moreover, in various alternative embodiments, a surgical instrument comprises a staple cartridge jaw is movable between an unclamped position and a clamped position relative to an anvil jaw. In any event, the handle 10100 comprises a lock configured to releasably hold the closure actuator 10140 in its clamped position. The handle 10100 further comprises release actuators 10180a, 10180b which, when either one is actuated, unlock the closure actuator 10140 such that the end effector can be re-opened. In various alternative embodiments, the handle 10100 comprises an electric motor configured to move the closure tube 10240 proximally and/or distally when actuated by the clinician.

The end effector 10400 is attached to the shaft 10200 about an articulation joint 10500 and is rotatable within a plane about an articulation axis. The shaft 10200 defines a longitudinal axis and the end effector 10400 is articulatable between a position in which the end effector 10400 is aligned with the longitudinal axis and positions in which the end effector 10400 extends at a transverse angle relative to the longitudinal axis. The handle 10100 comprises an electric motor and a control system configured to control the operation of the electric motor. The electric motor comprises a brushless DC motor; however, the electric motor can comprise any suitable motor, such as a brushed DC motor, for example. The entire disclosure of U.S. Patent No. 10,149,683 is relevant. The entire disclosure of U.S. Patent Application Publication No. 2018/0125481 is relevant. The handle 10100 further comprises a replaceable and/or rechargeable battery 10300 attachable to the handle housing which powers the surgical instrument 10000. The entire disclosure of U.S. Patent No. 8,632,525is relevant. The electric motor is operably coupled with a firing drive 10250 of the surgical instrument 10000 and is configured to drive a firing member of the firing drive 10250 through a staple firing stroke. The electric motor comprises a rotatable output including a gear engaged with a translatable rack of the firing drive 10250. The electric motor is operated in a first direction to drive the firing member through the staple firing stroke and a second, or opposite, direction to retract the firing member and/or reset the firing drive 10250. The surgical instrument 10000 further comprises an actuator 10150 in communication with the motor control system which, when actuated or rotated, signals to the motor control system to operate the electric motor in the first direction and begin the staple firing stroke. If the actuator 10150 is released, the motor control system stops the electric motor. When the actuator 10150 is re-actuated, the motor control system operates the electric motor in the first direction once again to continue the staple firing stroke. When the firing member reaches the end of the staple firing stroke, the control system stops the electric motor awaiting input from the clinician. When the clinician releases the actuator 10150 at such point, the control system reverses the operation of the electric motor to retract the firing member back into its unfired position. The handle 10100 further comprises a retraction actuator in communication with the motor control system that reverses the direction of the electric motor to retract the firing drive when actuated by the clinician. When the retraction actuator is depressed, the staple firing stroke is terminated regardless of whether the firing member had reached the end of the staple firing stroke.

The electric motor of the surgical instrument 10000 is also used to selectively drive an articulation drive system to articulate the end effector 10400. More specifically, the articulation drive system comprises an articulation driver that is selectively engageable with the firing drive and, when the articulation driver is engaged with the firing drive, the articulation driver is movable proximally and distally by the operation of the electric motor to articulate the end effector 10400. When the electric motor is operated in its first direction, in such instances, the end effector 10400 is articulated in a first direction to push the articulation driver distally. Similarly, the end effector 10400 is articulated in a second direction when the electric motor is operated in its second direction to pull the articulation driver proximally. When the articulation driver is not engaged with the firing drive, the operation of the electric motor does not articulate the end effector 10400. Instead, in such instances, the electric motor only moves the firing drive. That said, it should be appreciated that the movement of the firing drive to articulate the end effector 10400 does not cause the staple firing stroke to be performed. The range of motion needed to articulate the end effector 10400 is small, as compared to the range of motion of the staple firing stroke, and occurs proximal to the beginning of the staple firing stroke such that the staples are not ejected and the tissue is not cut while the end effector 10400 is being articulated. The surgical instrument 10000 further comprises an articulation lock which unlocks when the articulation driver is moved longitudinally by the firing drive and then locks the end effector 10400 in position when the articulation driver is not being driven by the firing drive. The entire disclosure of U.S. Patent No. 9,629,629is relevant. The above being said, a surgical instrument can comprise a separate articulation motor in addition to the firing motor for driving the articulation drive system.

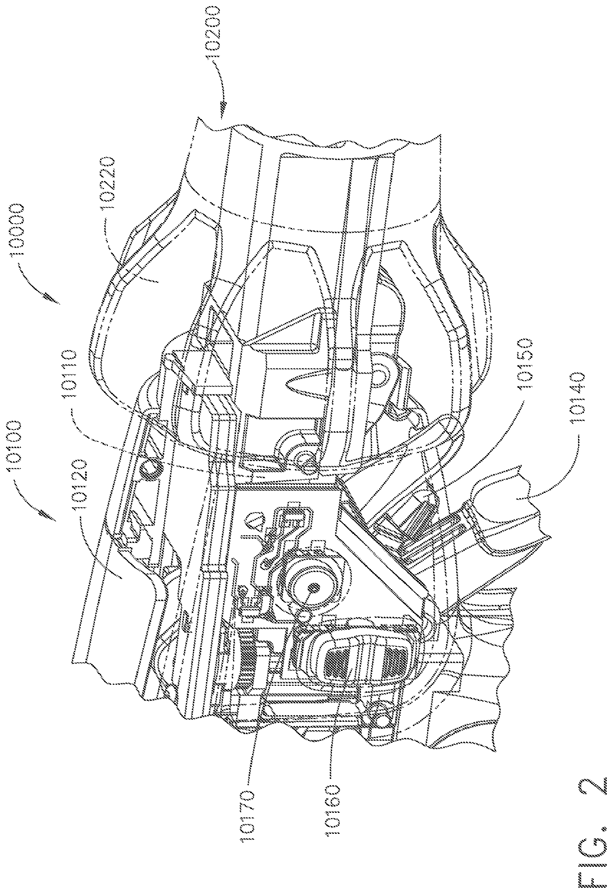

Further to the above, referring to FIG. 2, the handle 10100 comprises a frame 10110, a housing 10120, and an articulation actuator 10160. The articulation actuator 10160 comprises a rocker switch, for example, which is oriented vertically on the housing 10120 and is in communication with the motor control system. The rocker switch is rotatable upwardly and downwardly about an axis to articulate the end effector 10400. The upper portion of the articulation actuator 10160 is pushed by the clinician to articulate the end effector 10400 to the left and the lower portion of the articulation actuator 10160 is pushed to articulate the end effector 10400 to the right. Such an arrangement provides an intuitive interface for the clinician; however, any suitable arrangement could be used. The handle 10100 further comprises a home actuator 10170 in communication with the motor control system. When the home actuator 10170 is actuated by the clinician, the motor control system operates the electric motor to re-center the end effector 10400 along the longitudinal axis of the shaft 10200 of the surgical instrument 10000. To this end, the control system is configured to track the position of the end effector such that, when the home actuator 10170 is actuated, the control system operates the electric motor in the correct direction to articulate the end effector 10400 in the correct direction and the correct amount. In various instances, the surgical instrument 10000 comprises a linear encoder configured to track the position of the articulation driver, for example, such that, when the home actuator 10170 is actuated, the control system can properly center the end effector 10400.

Further to the above, the shaft 10200 is rotatable relative to the handle 10100. The shaft 10200 comprises a frame 10210 attached to the frame 10110 of the handle 10100. In embodiments where the shaft 10200 is readily removable from the handle 10100, the shaft frame 10210 can detach from the handle frame 10110. In embodiments where the shaft 10200 is not removable from the handle 10100, the shaft frame 10210 and the handle frame 10110 can be integrally formed. In any event, the shaft 10200 comprises a nozzle, or grip, 10220 fixedly mounted to the closure tube 10240 of the shaft 10200. The grip 10220 comprises finger grooves 10222 defined therein and ridges 10224 extending between the finger grooves 10222 that provide walls against which a clinician can push their finger and assist the clinician in rotating the shaft 10200 about its longitudinal axis.

Notably, further to the above, the end effector 10400 rotates with the shaft 10200 when the shaft 10200 is rotated about its longitudinal axis. Thus, the end effector 10400 rotates clockwise when the shaft 10200 is rotated clockwise by the clinician and counter-clockwise when the shaft 10200 is rotated counter-clockwise by the clinician. In various alternative embodiments, the surgical instrument 10000 comprises an electric motor configured to rotate the shaft 10200 about its longitudinal axis. In either event, the shaft 10200 is rotatable from a top-dead-center (TDC) position in which the anvil 10420 is positioned directly above the staple cartridge jaw 10410 to any other suitable position within a full 360 degree range of positions. For instance, the shaft 10200 is rotatable into a right 90 degree position in which the anvil 10420 is facing to the right of the handle 10100 or a left 90 degree position in which the anvil 10420 is facing to the left of the handle 10100. The shaft 10200 is also rotatable into a bottom-dead-center (BDC) position in which the staple cartridge jaw 10410 is positioned directly above the anvil 10420.

As described above, the end effector 10400 is both articulatable about the articulation joint 10500 and rotatable with the shaft 10200. When the end effector 10400 is rotated in a plane when the end effector 10400 is in its TDC position, as mentioned above, the articulation control 10160 is intuitive to the user - push up to articulate left and push down to articulate right. This arrangement is also intuitive even after the shaft 10200 - and end effector 10400 - have been rotated 90 degrees to the right or to the left. However, when the shaft 10200 and end effector 10400 have been rotated past 90 degrees in either direction, the articulation control 10160 can become counter-intuitive to the clinician. In fact, the articulation control 10160 can seem backwards. With this in mind, the control system of the surgical instrument 10000 is configured to flip the manner in which the surgical instrument responds to the articulation control 10160 when the shaft 10200 and end effector 10400 have been rotated past 90 degrees in either direction. In such instances, the controls become: push up to articulate right and push down to articulate left. To this end, as described in greater detail below, the surgical instrument 10000 is configured to detect the orientation of the shaft 10200 relative to the handle 10100, i.e., it is configured to detect whether the end effector 10400 is at least partially upside down with respect to the handle 10100 and then enter an alternative operational control mode in which the responsiveness of the surgical instrument 10000 to the articulation control 10160 has been reversed. Such an arrangement can make the surgical instrument 10000 easier to use in various instances.

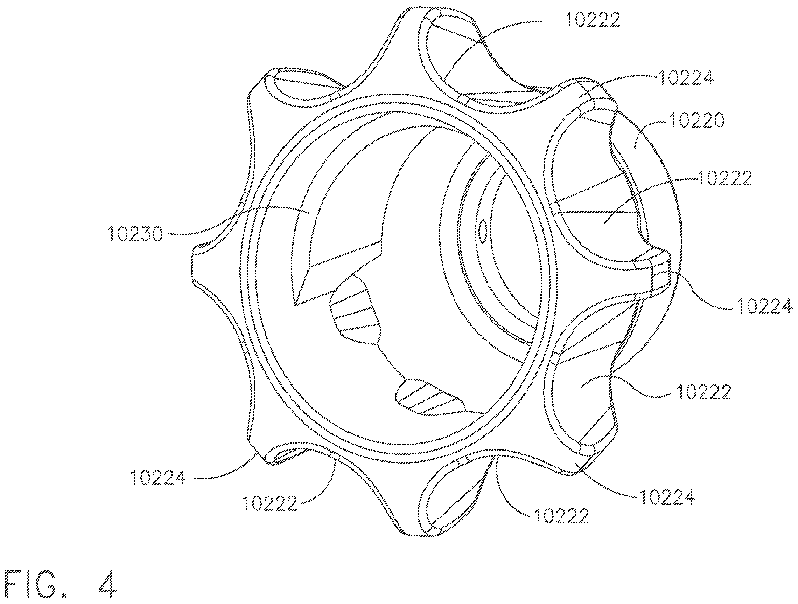

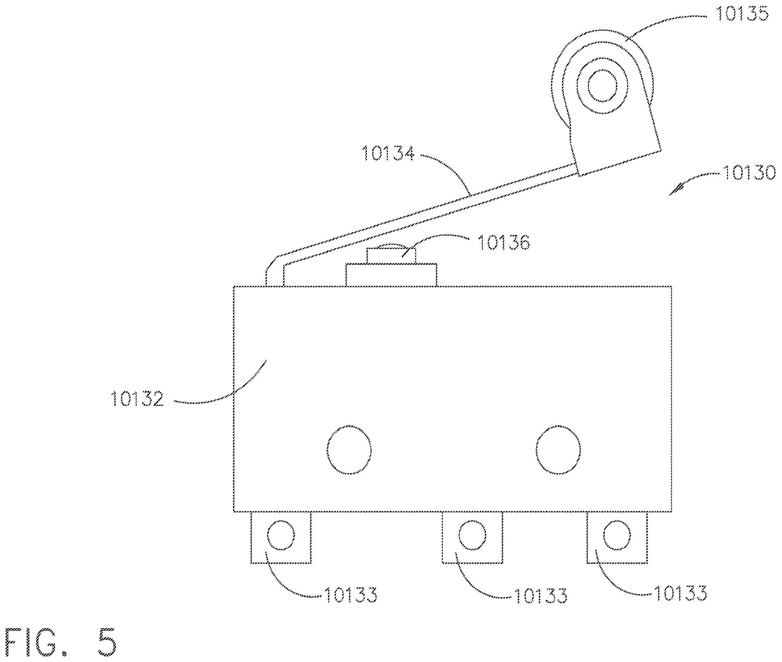

Referring to FIGS. 2-5, the surgical instrument 10000 comprises a switch 10130 mounted to the handle 10100 in communication with the control system which is configured to detect the rotation of the shaft 10200 relative to the handle 10100. The switch 10130 comprises a switch body 10132 fixedly mounted to the handle frame 10110 and three electrical contacts 10133 which are part of a switch circuit in communication with the control system. The switch 10000 further comprises a switch arm 10134 rotatably connected to the switch body 10132 and an electrical contact 10136 positioned on the switch body 10132. The switch arm 10134 is comprised of an electrically-conductive material, such as brass, for example, and closes the switch circuit when the switch arm 10134 comes into contact with the electrical contact 10136. The switch arm 10134 is rotated between an open position (FIG. 5) and a closed position when the shaft 10200 is rotated past the left or right 90 degree positions. More specifically, the grip, or nozzle, 10220 comprises a cam 10230 defined thereon which pushes the switch arm 10134 into its closed position when the shaft 10200 and the end effector 10400 is at least partially upside down. When the shaft 10200 is rotated upwardly past the 90 degree positions, the cam 10230 permits the switch arm 10134 to resiliently move back into its open position and open the switch circuit. The switch arm 10134 comprises a roller 10135 mounted thereto to facilitate relative rotation between the switch arm 10134 and the grip 10220.

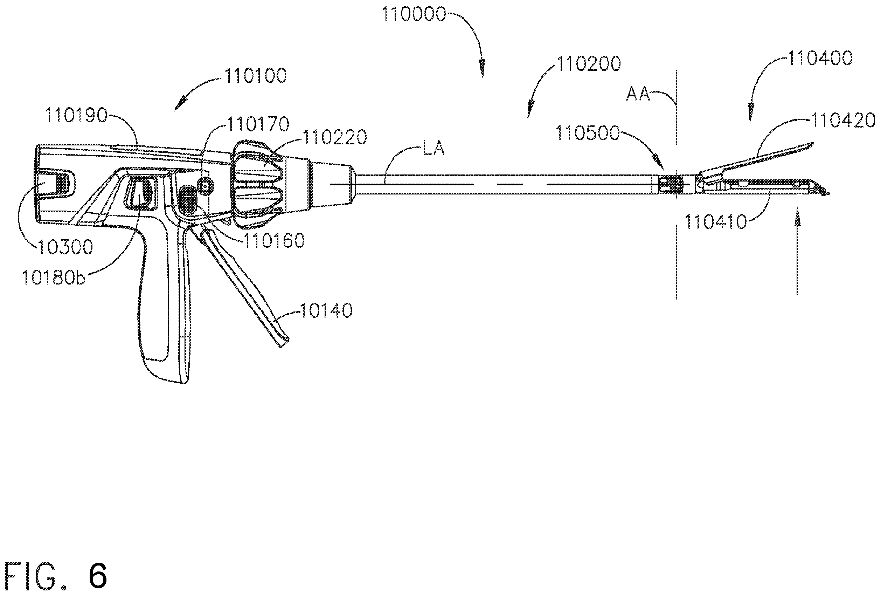

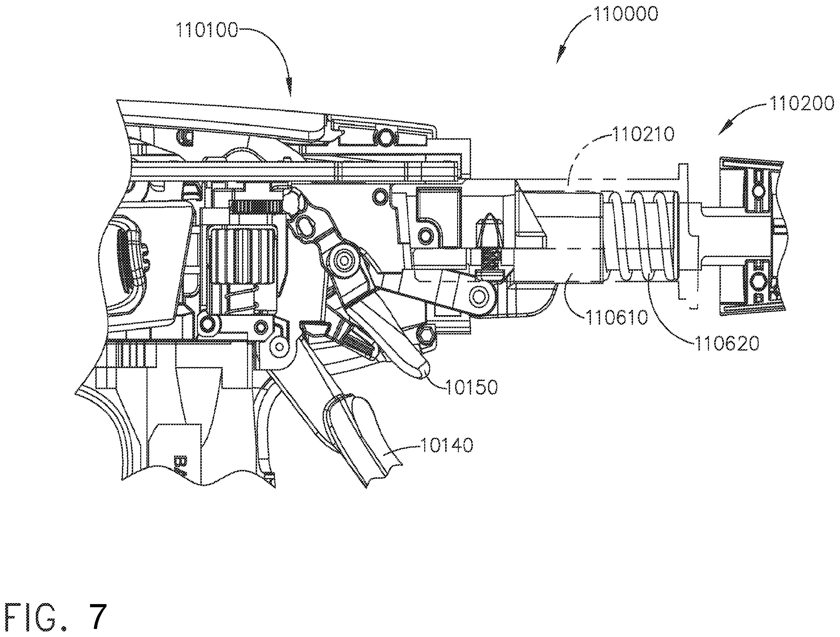

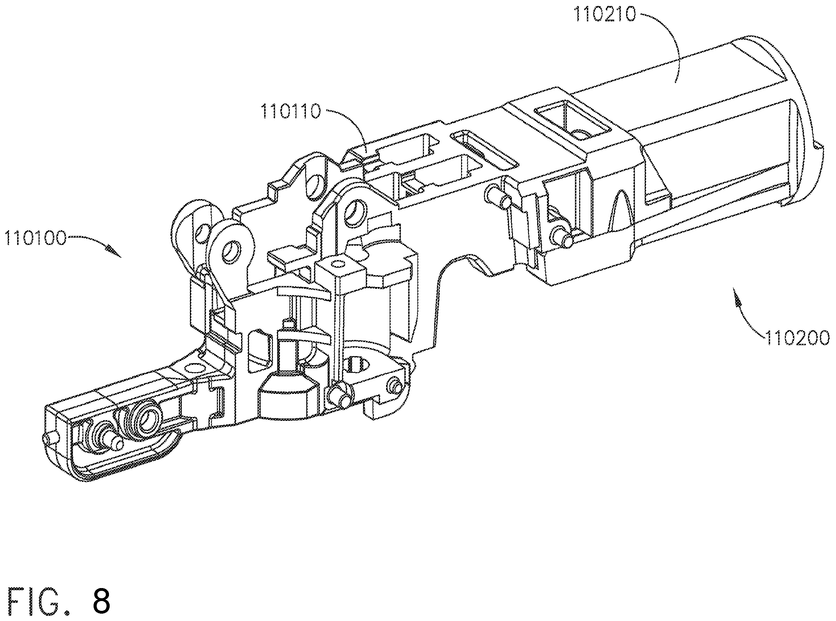

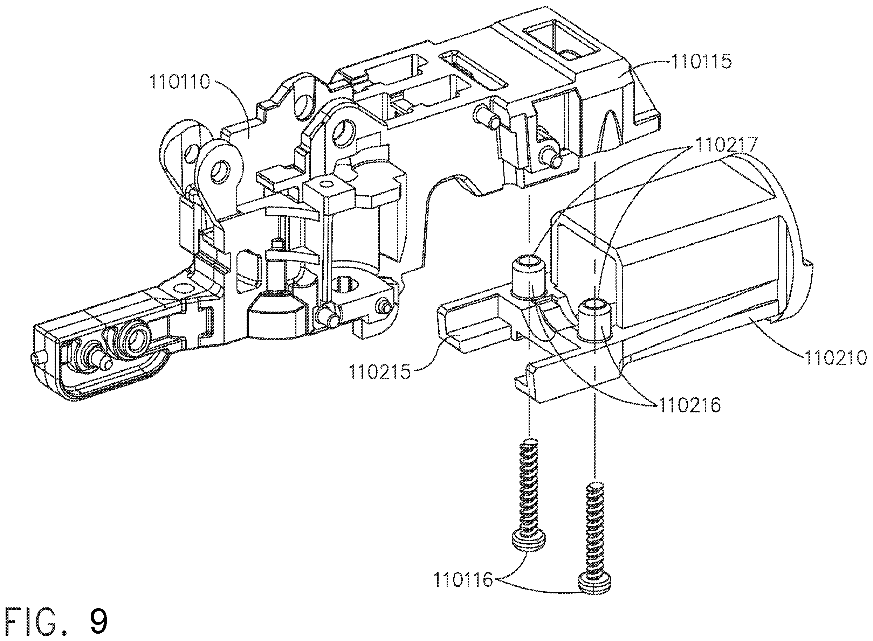

A surgical instrument 110000 is illustrated in FIG. 6. The surgical instrument 110000 comprises a handle 110100, a shaft 110200 extending from the handle 110100, and an end effector 110400 rotatably connected to the shaft 110200 about an articulation joint 110500. The surgical instrument 110000 is similar to the other surgical instruments disclosed herein and such similarities are not discussed herein for the sake of brevity. The shaft 110200 is fixedly attached to the handle 110100. Referring to FIGS. 7-9, the handle 110100 comprises a handle frame 110110 and the shaft 110200 comprises a shaft frame 110210. The handle frame 110110 comprises a distal portion 110115 which extends over and nests with a proximal portion 110215 of the shaft frame 110210. The shaft frame 110210 comprises alignment projections 110216 extending therefrom which are closely received within apertures defined in the handle frame 110110. Each of the projections 110216 comprises an aperture 110217 defined therethrough which is configured to receive a self-tapping screw 110116, for example. The self-tapping screws are configured to gain purchase into the handle frame 110115 and fixedly secure the shaft 110200 to the handle 110100. In various instances, referring again to FIG. 6, a force can be applied to the end effector 110400 to dislodge a staple cartridge positioned therein without creating relative movement between the shaft 110200 and the handle 110100.

Further to the above, the surgical instrument 110100 comprises an articulation drive which is actuatable to articulate the end effector 110400 about an articulation axis AA, a closure drive including a closure actuator 10140, described above, which is actuatable to move a jaw 110420 of the end effector 110400 toward a jaw 110410, and a staple firing drive which is actuatable to fire the staples from the staple cartridge seated in the end effector 110400 during a staple firing stroke. The staple firing drive comprises an electric motor configured to advance a firing member distally through the staple firing stroke and retract the firing member proximally back into its unfired position. Similar to other embodiments described herein, the articulation drive is selectively engageable with the staple firing drive. An articulation member of the articulation drive is driveable by the staple firing drive when the articulation drive is engaged with the staple firing drive and, correspondingly, the articulation drive is not driveable by the staple firing drive when the articulation drive is not engaged with the staple firing drive. As described further below, the closure drive decouples the articulation drive from the staple firing drive when the closure drive is sufficiently actuated.

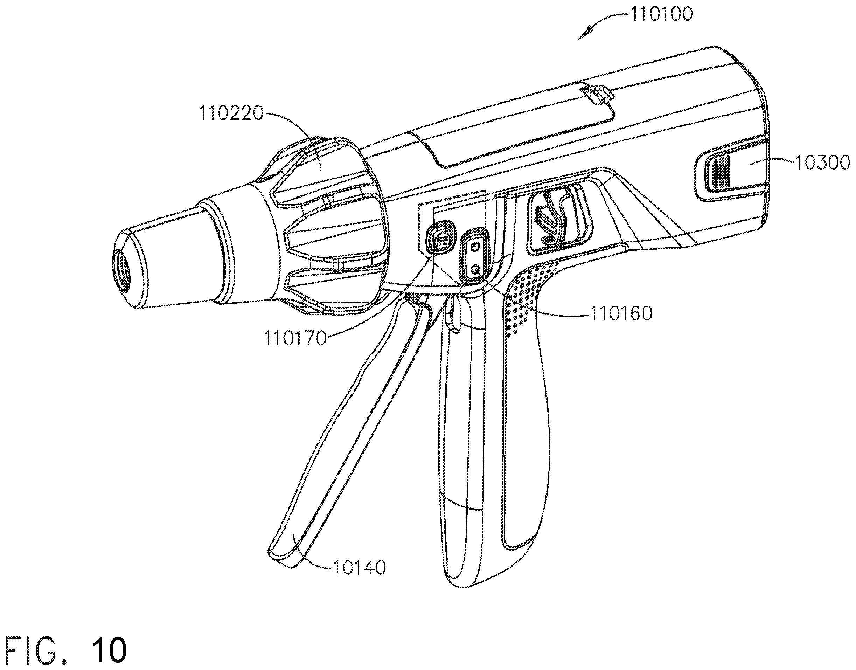

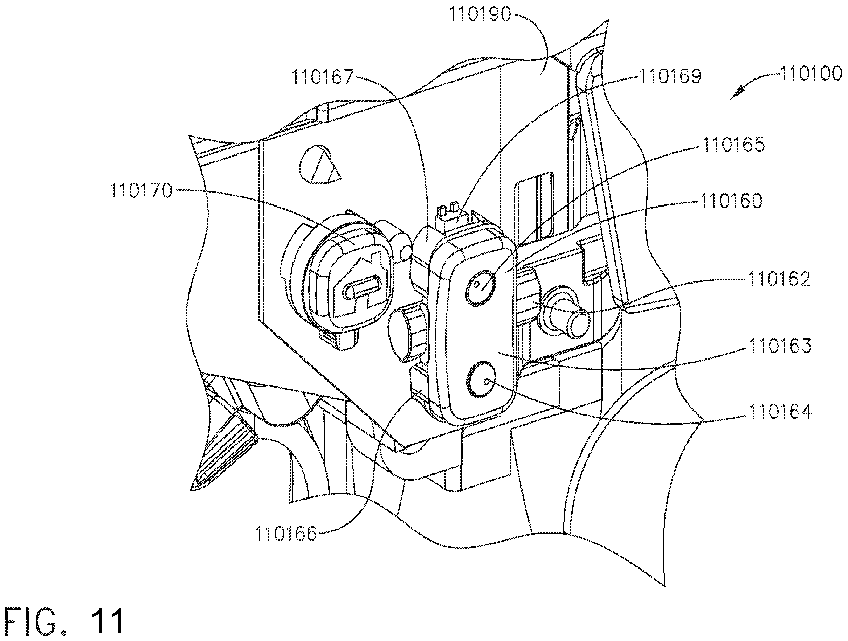

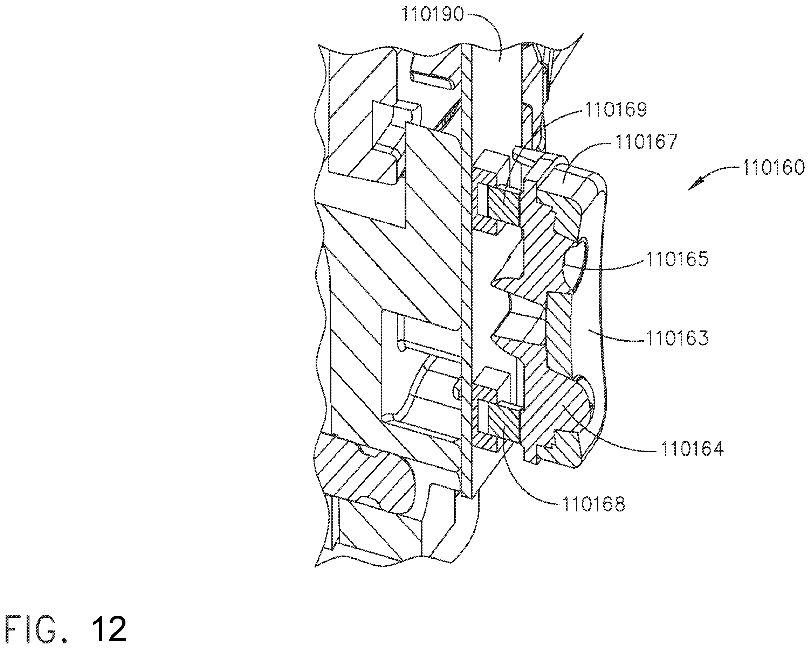

Referring to FIGS. 10-12, the handle 110100 comprises an articulation actuator 110160 which is actuatable to articulate the end effector 110400. The articulation actuator 110160 comprises a rocker switch, for example, including a rocker body 110163 which is rotatably mounted to a circuit board 110190 about a pivot 110162. The articulation actuator 110160 further comprises a first contact 110168 mounted to the circuit board 110190 which is moved from an open state to a closed state when a first end 110164 of the rocker body 110163 is depressed. When the first end 110164 is released, a biasing member in the first contact 110168 returns the first contact back into its open state. The articulation actuator 110160 also comprises a second contact 110169 mounted to the circuit board 110190 which is moved from an open state to a closed state when a second end 110165 of the rocker body 110163 is depressed. When the second end 110165 is released, a biasing member in the second contact 110169 returns the second contact back into its open state. The first contact 110168 and the second contact 110169 are in communication with a control system of the surgical instrument 110000. When the control system detects that the first contact 110168 has been closed, the control system operates the electric motor of the staple firing system to articulate the end effector 110400 in a first direction. Correspondingly, the control system operates the electric motor of the staple firing system to articulate the end effector 110400 in a second direction when the control system detects that the second contact 110169 has been closed.

Further to the above, the rocker body 110163 comprises a first stand-off 110166 that contacts the circuit board 110190 when the rocker body 110163 is depressed in the first direction and limits the travel of the rocker body 110163. Similarly, the rocker body 110163 comprises a second stand-off 110167 that contacts the circuit board 110190 when the rocker body 110163 is depressed in the second direction and limits the travel of the rocker body 110163. Such an arrangement prevents or reduces the possibility of the articulation actuator 110160 from being damaged. Such an arrangement can also be adapted to other actuators on the handle 110100, such as an actuator 110170, for example. The actuator 110170 comprises a switch in communication with the control system of the surgical instrument 110100 which, when closed, causes the control system to automatically re-center the end effector 110400 along a longitudinal axis LA (FIG. 6) of the shaft 110200.

Further to the above, the shaft 110200 and the end effector 110400 are rotatable relative to the handle 110100 about the longitudinal axis LA. In use, a clinician can grasp a nozzle-shaped portion, or nozzle, 110220 of the shaft 110200 to rotate the shaft 110200 about the longitudinal axis.

In various embodiments, further to the above, the control system of the surgical instrument 110000 becomes unresponsive to the articulation actuators 110160 and 110170 when the closure trigger 10140 is initially actuated to close the end effector 110400. Moreover, in such embodiments, the initial actuation of the closure trigger 10140 causes the articulation drive to decouple from the staple firing drive. Such embodiments entirely avoid the possibility of the end effector 110400 articulating while the end effector 110400 is clamped onto the tissue. That said, such embodiments require the clinician to estimate where the second jaw 110420 will contact the tissue when the second jaw 110420 is eventually closed after the end effector 110400 has been articulated. If the clinician has already partially-closed the end effector 110400, in such embodiments, the clinician must re-open the end effector 110400 to re-articulate the end effector 110400. In such embodiments, re-opening the end effector 110400 re-engages the articulation drive with the staple firing drive and the control system becomes responsive once again to the articulation actuators 110160 and 110170. In alternative embodiments, the end effector 110400 of the surgical instrument 110000 can be articulated while the end effector 110400 is in a partially-closed, or partially-clamped, configuration. Once the end effector 110400 is closed more than the partially-closed configuration, in these embodiments, the articulation drive is decoupled from the staple firing drive and the control system is no longer responsive to the articulation controls 110160 and 110170 until the end effector 110400 is re-opened or at least returned back to its partially-closed configuration.

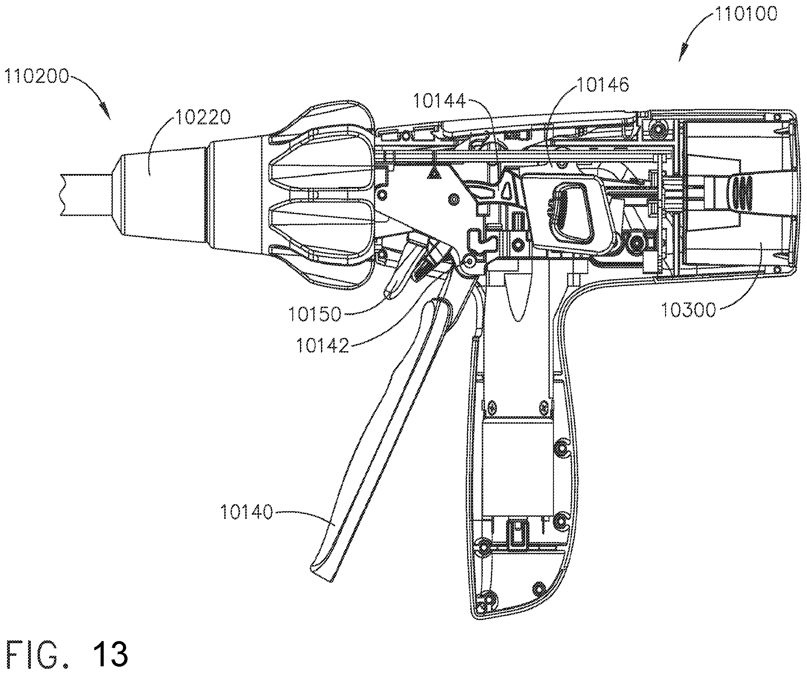

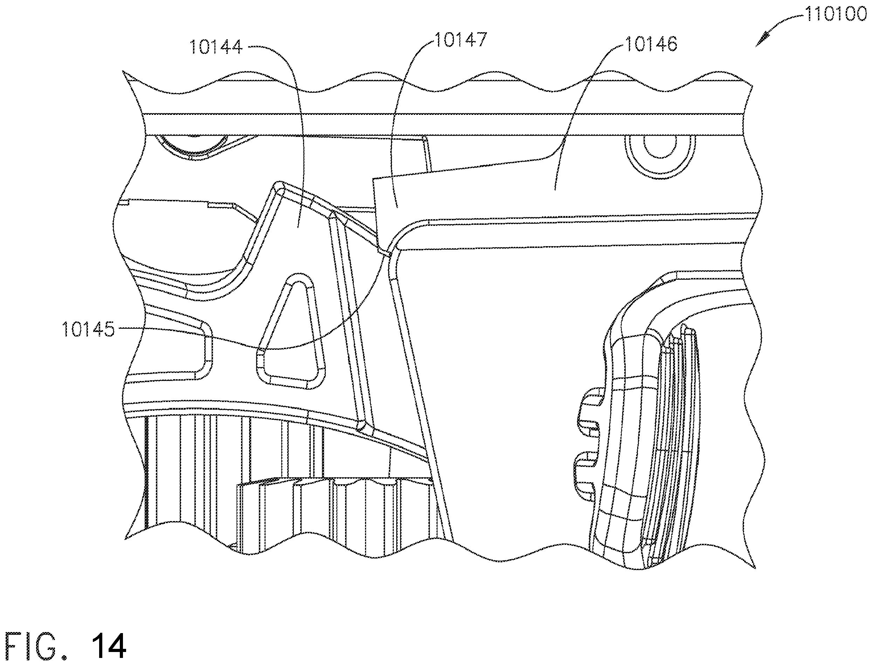

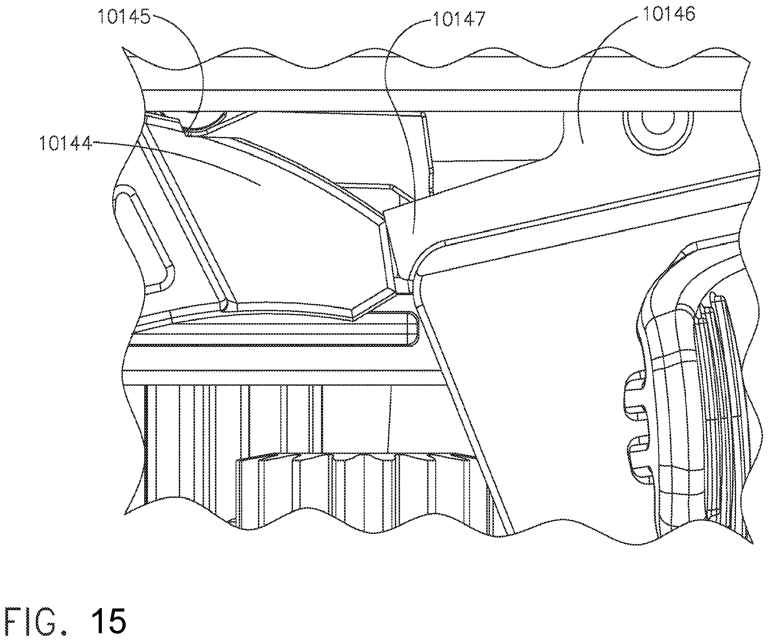

Further to the above, the partially-closed configuration of the end effector 110400 is a predefined, or predetermined, position of the second jaw 110420. In at least one such embodiment, referring to FIGS. 13-15, the surgical instrument 110100 comprises a closure lock 10146 configured to releasably hold the closure actuator 10140 in the pre-defined, partially-closed position. When the closure actuator 10140 is in this partially-closed position, the articulation drive is still engaged with the staple firing drive and the control system is responsive to the articulation controls 110160 and 110170. Stated another way, the articulation drive is engaged with the staple firing drive and the control system is responsive to the articulation controls 110160 and 110170 when the closure actuator 10140 is in a position between, and including, the open position and the pre-defined, partially-closed position. FIG. 14 illustrates a lock arm 10147 of the closure lock 10146 seated in a notch, or recess, 10145 defined in a top portion 10144 of the closure actuator 10140. The lock arm 10147 engages the notch 10145 as the closure actuator 10140 is being closed, i.e., when the closure actuator 10140 reaches the partially-closed position discussed above. In various instances, the lock arm 10147 entering the notch 10145 can make an audible click which can indicate to the clinician closing the closure actuator 10140 that any additional closure of the closure actuator 10140 will disable the articulation drive and controls. The lock arm 10147 entering into the notch 10145 can also provide a tactile feedback to the clinician. At such point, the clinician is afforded an opportunity to observe the articulated position of the end effector 110400 and the partially-closed configuration of the end effector 110400 while the closure actuator 10140 is held in position. If the clinician is unsatisfied with the position of the end effector 110400 in this instance, the clinician is afforded an opportunity to articulate the end effector 110400 once again using the articulation controls 110160 and 110170 without having to re-open the end effector 110400. Closing the closure actuator 10140 beyond this position, however, decouples the articulation drive from the staple firing drive and makes the control system unresponsive to the articulation controls 110160 and 110170. In such instances, the lock arm 10147 flexes out of engagement with the notch 10145 such that the top portion 10144 rotates past the lock arm 10147 until the closure actuator 10140 reaches the end of its stroke. At such point, referring to FIG. 15, the lock arm 10147 unflexes and falls in behind the top portion 10144 to releasably hold the closure actuator 10140 in its fully-closed position. Applying a force to the closure actuator 10140 can flex the lock arm 10147 out of the way once again so as to return the closure actuator 10140 to its above-discussed partially-closed position and/or fully-open position. When the closure actuator 10140 is returned to the partially-closed position, and/or anywhere in-between the partially-closed position and the open position, the articulation drive is re-engaged with the staple firing drive and the control system is once again responsive to the articulation controls 110160 and 110170.

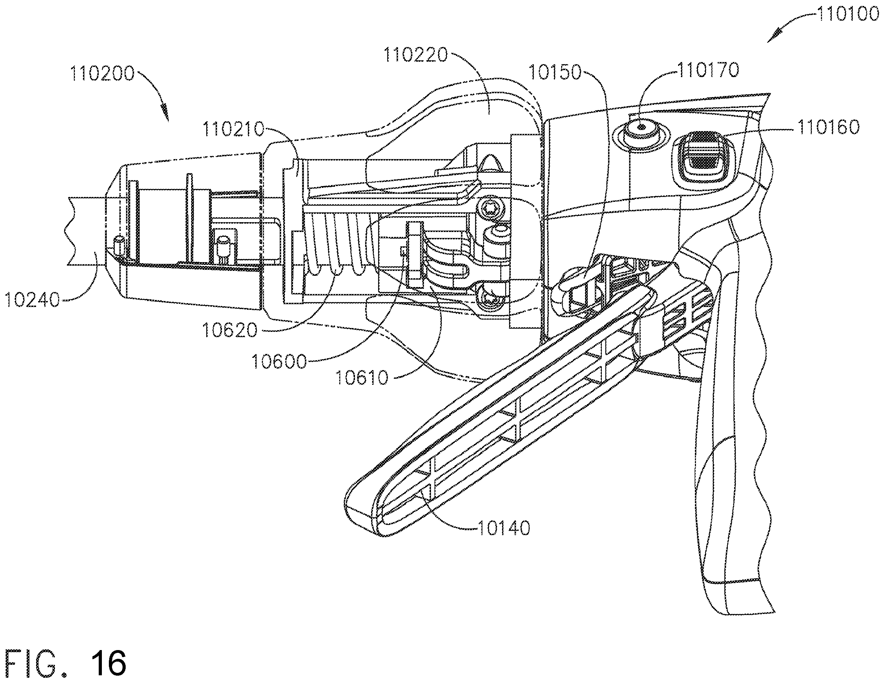

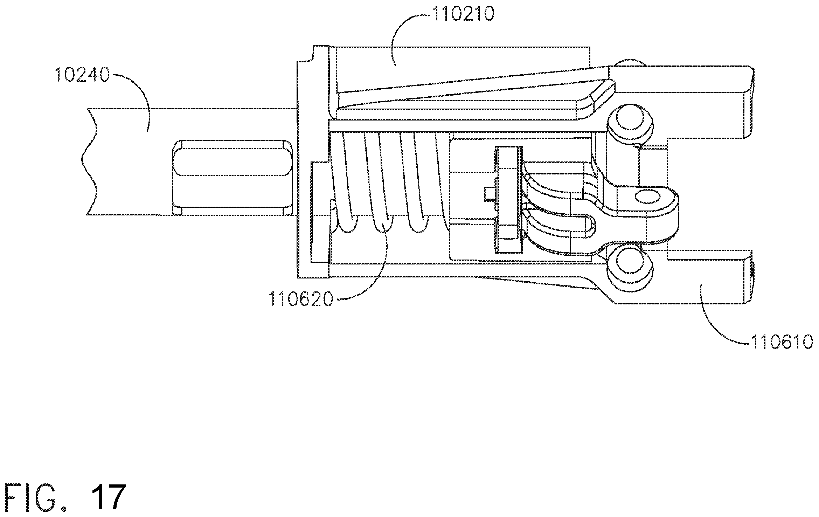

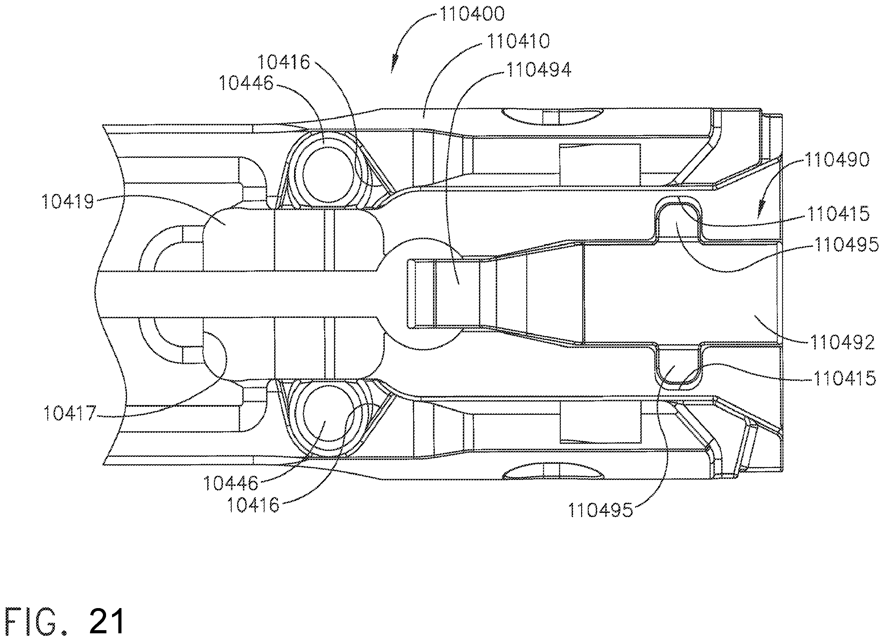

When the closure actuator 10140 is closed, referring now to FIGS. 16 and 17, the closure actuator 10140 drives a closure drive 10600 to close the second jaw 110420 of the end effector 110400. The closure drive 10600 includes a carriage 110610 which is pushed distally by the top portion 10144 of the closure actuator 10140 as the closure actuator 10140 is moved into its closed position by the clinician. The closure drive 10600 further includes a closure tube assembly 10240 mounted to the carriage 110610 which moves distally with the carriage 110610. The closure tube assembly 10240 comprises a distal end which interfaces with the second jaw 110420 and moves the second jaw 110420 downwardly toward the first jaw 110410 as the closure tube assembly 10240 is advanced distally. The closure drive 10600 also includes a spring 110620 positioned intermediate the carriage 110610 and the shaft frame 110210 which is resiliently compressed between the carriage 110610 and the shaft frame 110210 as the carriage 110610 is advanced distally during the closure stroke. After the closure stroke is completed, the spring 110620 is held in its compressed state by the closure lock 10146 (FIG. 14), discussed above, until the closure lock 10146 is overcome by an opening force provided by opening actuators 10180a and 10180b (FIG. 6) on the handle 110100. At such point, the compressed spring 110620 pushes the carriage 110610 and the closure tube assembly 10240 proximally to re-position the closure actuator 10140 in its unactuated position and to permit jaw opening springs 10446 (FIG. 21) in the end effector 110400 to open the second jaw 110420.

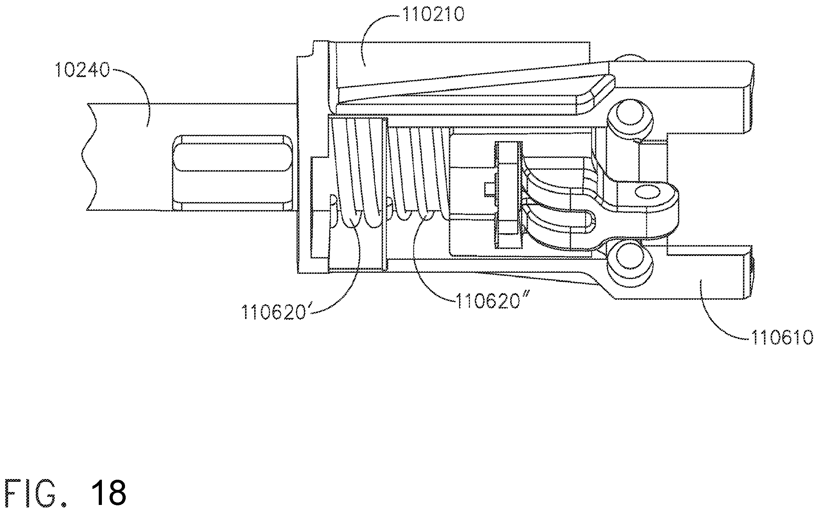

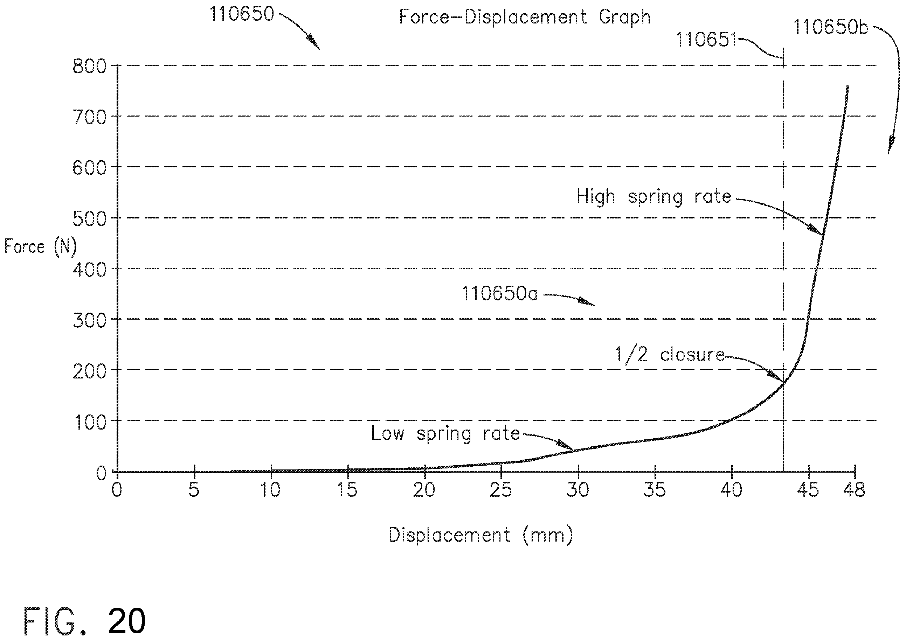

In various alternative embodiments, further to the above, a closure drive can include more than one spring that is compressed between the closure carriage 110610 and the shaft frame 110210. Referring to FIG. 18, a closure drive can comprise a distal spring 110620' and a proximal spring 110620" in series with one another. The distal spring 110620' is stiffer than the proximal spring 110620" such that the proximal spring 110620" is compressed significantly before the distal spring 110620' compresses significantly. As a result, the initial movement of the closure actuator 10140 from its fully-open position will encounter a light force owing to the compression of the proximal spring 110620" that suddenly increases once the distal spring 110620' begins to compress significantly. In at least one such instance, the proximal spring 110620" reaches its fully-compressed, or solid, state before the distal spring 110620' begins to compress significantly. This sudden increase in the force being applied to the closure actuator 10140 can correspond to the point in the closure stroke in which the articulation system has been deactivated. In such instances, the clinician is provided with tactile feedback that the articulation system can no longer be used to articulate the end effector 110400 unless the closure actuator 110400 is at least partially released, or re-opened, back beyond the force transition point. A graphical representation of the force applied to the closure actuator 10140 by the springs 110620' and 110620" is depicted in FIG. 20. The force applied to the closure actuator 10140 is depicted by line 110650 which includes an initial portion 110650a and a final portion 110650b. In the initial portion 110650a, as outlined above, the proximal spring 110620" compresses easily during the initial portion of the closing stroke resulting in a low force, around 100 N, being applied to the closure actuator 10140. At the half-way point in the closure stroke, for example, the force applied to the closure actuator 10140 in the final portion 110650b increases significantly owing to the solid state of the proximal spring 110620" and the higher spring rate of the distal spring 110620'. This force transition is demarcated as datum 110651 in FIG. 20 which also demarcates the deactivation of the articulation system.

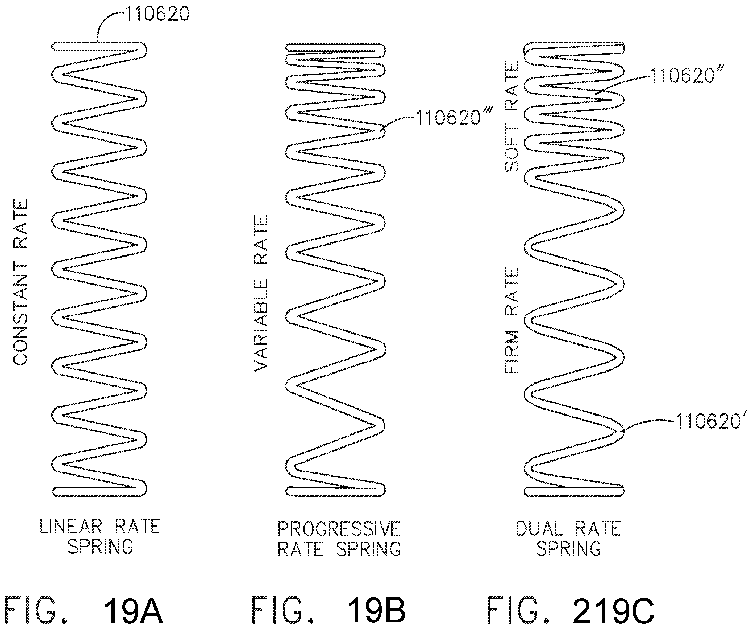

Further to the above, FIG. 19A depicts the spring 110620, discussed above, which has a constant spring rate along the length thereof. FIG. 19C is a graphical representation of a spring system including the distal spring 110620' and the proximal spring 110620" which have different spring rates. In various instances, the effect provided by the distal spring 110620' and the proximal spring 110620" can be combined into a single spring, such as spring 110620‴ in FIG. 19B, for example. In at least one embodiment, the spring 110620‴ comprises a spring rate which changes along the length thereof. In various embodiments, springs positioned intermediate the closure carriage 110610 and the shaft frame 110210 can comprise a parallel and/or series arrangement. Regardless of the spring arrangement used, the spring arrangement can provide a tactile feedback to the clinician that an operational transition or threshold has been crossed.

When the closure drive is advanced distally during the closure stroke, further to the above, the closure tube 110240 is advanced distally to engage and close the second jaw 110420 of the end effector 110400.

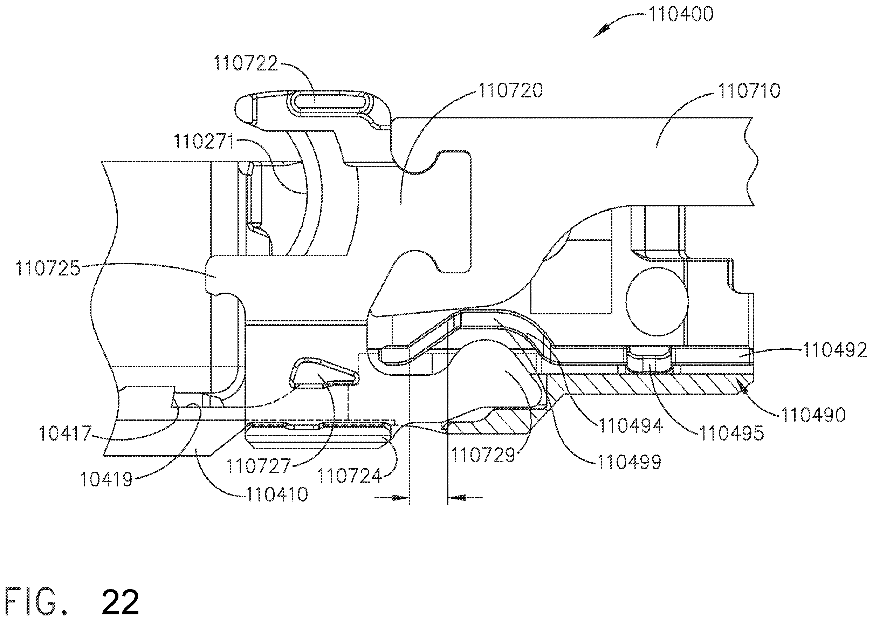

Once the end effector 110400 has been sufficiently closed, further to the above, the staple firing drive of the surgical instrument 110000 can be actuated to fire the staples contained in the staple cartridge seated in the end effector 110400 during a staple firing stroke. Referring to FIG. 22, the staple firing drive comprises a firing member, or bar, 110710 which is advanced distally by the electric motor of the staple firing drive in response to an actuation of the firing trigger 10150 (FIG. 7). The staple firing drive further comprises a coupling element 110720 attached to the distal end of the firing bar 110710. In at least one embodiment, the interface between the firing bar 110710 and the coupling element 110720 comprises a dovetail arrangement, for example. The coupling element 110720 is moveable between a proximal unfired position, illustrated in FIG. 22, and a distal fired position during a staple firing stroke. The coupling element 110720 comprises a cam 110724 configured to engage the first jaw 110410 and a cam 110722 configured to engage the second jaw 110420 during the staple firing stroke and hold the second jaw 110420. The cams 110722 and 110724 cooperate to hold the second jaw 110420 in position relative to the first jaw 110410 during the staple firing stroke, although embodiments are envisioned without the cams 110722 and 110724. The coupling element 110720 further comprises a tissue cutting edge 110271 configured to transect the tissue captured between the first jaw 110410 and the second jaw 110420 during the staple firing stroke.

Further to the above, the surgical instrument 110000 comprises a staple firing lockout to prevent the staple firing stroke when a staple cartridge is missing from the first jaw 110410 and/or when the staple cartridge seated in the first jaw 110410 has already been at least partially fired. To this end, the coupling element 110720 further comprises a proximally-extending tail 110729 which is biased downwardly, i.e., toward the bottom of the first jaw 110410, at the beginning of the staple firing stroke by a firing lockout spring 110490 mounted in the shaft 110200. Prior to the beginning of the firing stroke, referring to FIG. 22, the lockout spring 110490 is in an initially-unloaded state. In other words, the lockout spring 110490 does not apply any force to the proximally-extending tail 110729 of the coupling element 110720 when the coupling element 110720 is in its unfired position. Once the coupling element 110720 is advanced distally at the beginning of the staple firing stroke, the tail 110729 comes into contact with the lockout spring 110490 such that the lockout spring 110490 applies a downward force to the coupling element 110720. As discussed below, the downward force applied to the coupling element 110720 can, in various instances, lockout the staple firing drive and prevent the coupling element 110720 from being moved distally through the staple firing stroke. Such a configuration can avoid a continuous, and unnecessary, stress experienced by a firing assembly over time by only applying a lockout force to the firing assembly during the beginning of a firing stroke instead of applying a lockout force for an indefinite amount of time prior to the beginning of the firing stroke.

If an unfired staple cartridge is not seated in the first jaw 110410 at the beginning of the staple firing stroke, the firing lockout spring 110490 will push the coupling element 110720 downwardly such that a laterally-extending lock shoulder 110727 extending from the coupling element 110720 enters into a lock recess 10419 defined in the first jaw 10410 and contacts a lock shoulder 10417 at the distal end of the lock recess 10419 which blocks the distal advancement of the staple firing drive to prevent the staple firing stroke. If an unfired staple cartridge is seated in the first jaw 110410 at the beginning of the staple firing stroke, a distal end 110725 of the coupling element 110720 is supported by a sled in the staple cartridge which prevents the coupling element 110720 from being pushed into the lock recess 10419 by the firing lockout spring 110490 and, as a result, the coupling element 110720 can be advanced distally to perform the staple firing stroke. As the coupling element 110720 is being advanced distally through the staple firing stroke, the tail 110729 is no longer in contact with the firing lockout spring 110490 and, thus, the downward biasing force is not applied to the staple firing drive through the staple firing stroke. When the coupling element 117020 is retracted proximally after the staple firing stroke, the tail 110729 re-engages the lockout spring 110490 and deflects the lockout spring 110490 upwardly such that the tail 110729 can slide under the lockout spring 110490 back into its unfired position.

Further to the above, the firing lockout spring 110490 comprises a proximal portion 110492 mounted to the shaft 110200 and a distal end 110494 which is free to move relative to the proximal portion 110492. The distal end 110494 comprises an arcuate portion 110499 which extends over the proximal tail 110729 which is contacted by the proximal tail 110729 when the staple firing drive is actuated. The firing lockout spring 110490 further comprises lateral supports 110495 extending therefrom which supports the distal end 110494 over the proximal tail 110729. The lateral supports 110495 are positioned within recesses 110415 defined in the first jaw 110410 which hold the lateral supports 110495 in position. As a result of this arrangement, the firing lockout spring 110490 is prevented from bottoming out on the first jaw 110410 and shortening the effective length of the firing lockout spring 110490. Moreover, as a result of this arrangement, the firing lockout spring 110490 is able to flex and/or move upwardly to permit the coupling member 110720 to pass thereby without yielding or permanently deforming the firing lockout spring 110490. When the coupling member 110720 is returned to its proximal unfired position after the staple firing stroke, the firing lockout spring 110490 is moved upwardly by the coupling member 110720 to permit the coupling member tail 110729 to move thereunder.

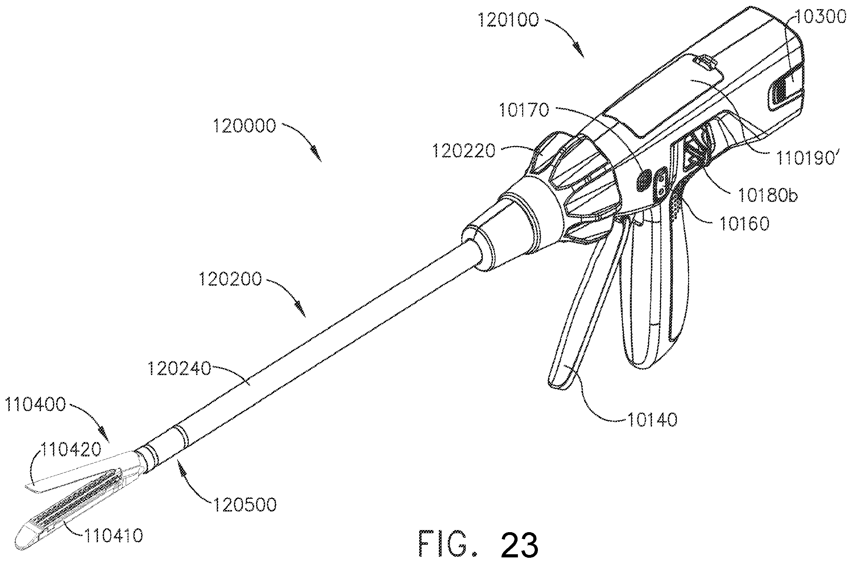

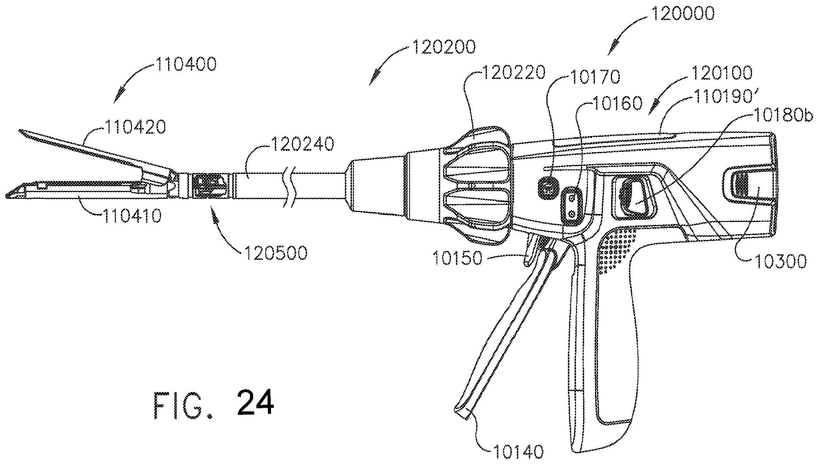

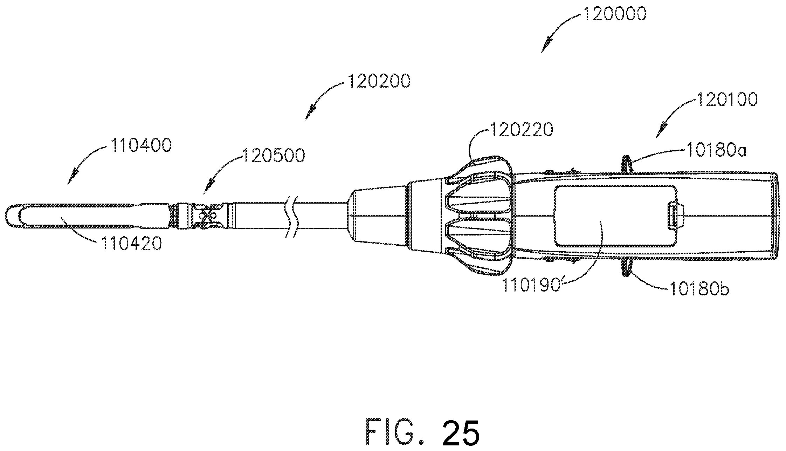

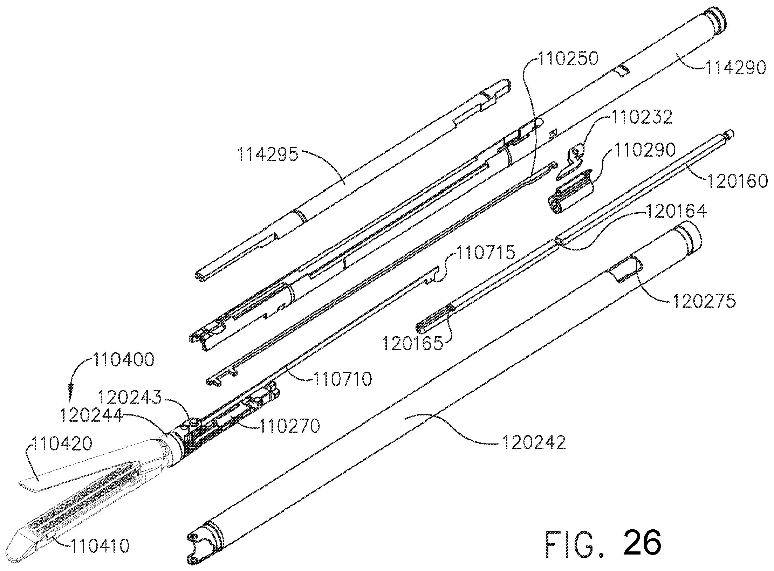

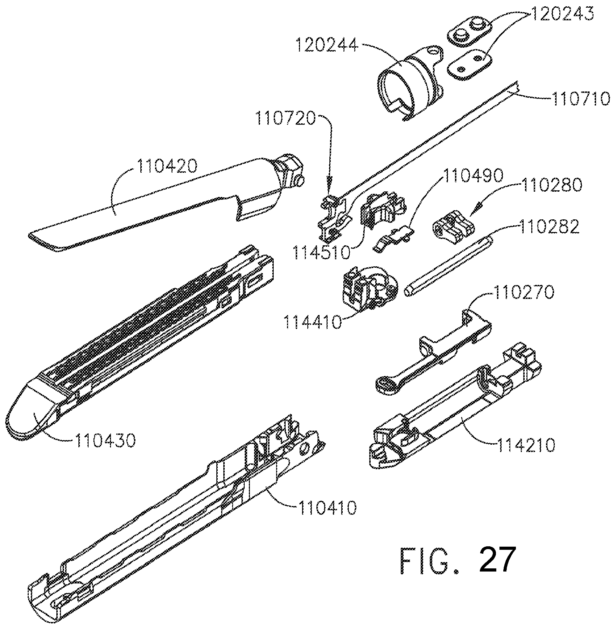

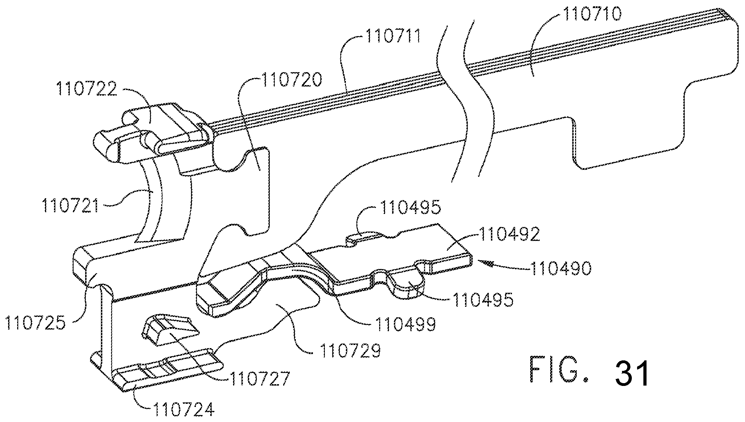

A surgical instrument 120000 is illustrated in FIGS. 23-31 and is similar to the other surgical instruments disclosed herein. The surgical instrument 120000 comprises a handle 120100, a shaft 120200 extending from the handle 120100, and an end effector 110400 rotatably coupled to the shaft 120200 about an articulation joint 120500. The handle 120100 comprises a frame, an outer housing 120110, and controls for operating the surgical instrument. Further to the above the handle 120100 comprises a drive system 120900 including an electric motor 120910 that is actuatable to articulate the end effector 120500, among other things. Referring to FIGS. 26 and 27, the firing drive 120190 further comprises a firing rod 120160 mounted to the firing rack 120150 such that the firing rod 120160 translates with the firing rack 120150. The firing drive 120190 also comprises a firing bar 110710 coupled to the firing rod 120160. Referring to FIG. 31, the firing bar 110710 comprises a plurality of flexible members, or laminates, 110711 that extend through the articulation joint 120500 which accommodate the articulation of the end effector 110400. As discussed further below, the firing drive 120900 is operable to articulate the end effector 110400 and, in a separate operating mode, drive the firing bar 110710 distally to perform a staple firing stroke.

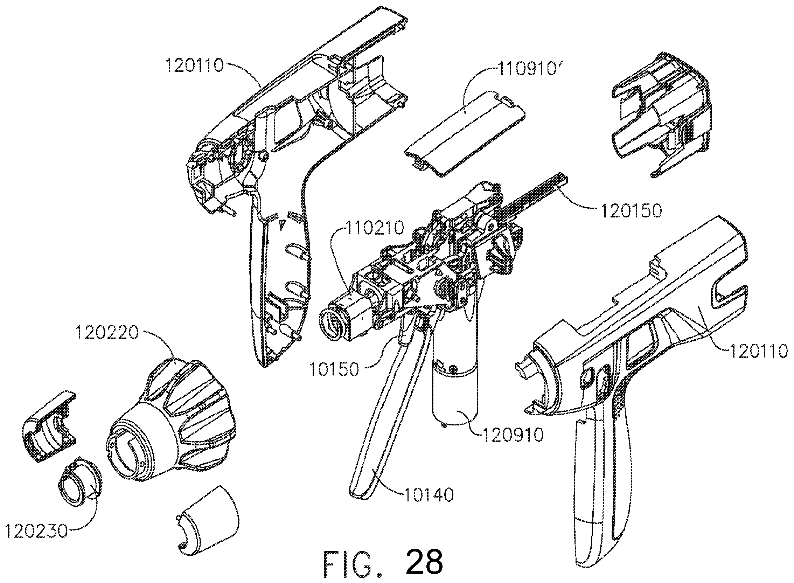

Further to the above, the end effector 110400 comprises a cartridge jaw 110410 and an anvil jaw 110420 rotatable relative to the cartridge jaw 110410 between an open position and a closed position. The surgical instrument 120000 further comprises a closure drive that is actuated by a closure trigger 10140 to move the anvil jaw 110420 into its closed position. More specifically, the closure trigger 10140 comprises a crank that pushes a closure tube 120240 of the shaft 120200 distally which contacts the anvil jaw 110420 and pushes the anvil jaw 110420 into its closed position. In alternative embodiments, the actuation of the closure trigger 10140 can operate an electric motor which drives the closure tube 120240. That said, embodiments having a mechanical coupling between the closure trigger 10140 and the closure tube 120240 provides the clinician with a feel of the clamping force being applied to the patient tissue while, on the other hand, a motor-driven system can assist the clinician with overcoming high clamping loads. In either event, the closure tube 120240 extends across the articulation joint 120500 and comprises a joint arrangement which accommodates the articulation of the end effector 110400 about the articulation joint 120500. Referring to FIGS. 27 and 28, the closure tube 120240 includes a proximal portion 120242 engaged with a closure carriage slideable within the handle 120100, a distal portion 120244 positioned on a distal side of the articulation joint 120500, and links 120243 rotatably connecting the distal portion 120244 to the proximal portion 120242. The links 120243 extend across and are generally aligned with the articulation joint 120500 which permit the closure tube 120240 to slide distally regardless of the articulated orientation of the end effector 110400.

Referring again to FIG. 31, the firing bar 110710 further comprises a distal knife head 110720 including a tissue knife edge 110721 configured to cut patient tissue during the staple firing stroke, a cam 110722 configured to engage the anvil jaw 110420 during the staple firing stroke, and a cam 110724 configured to engage the cartridge jaw 110410 during the staple firing stroke.

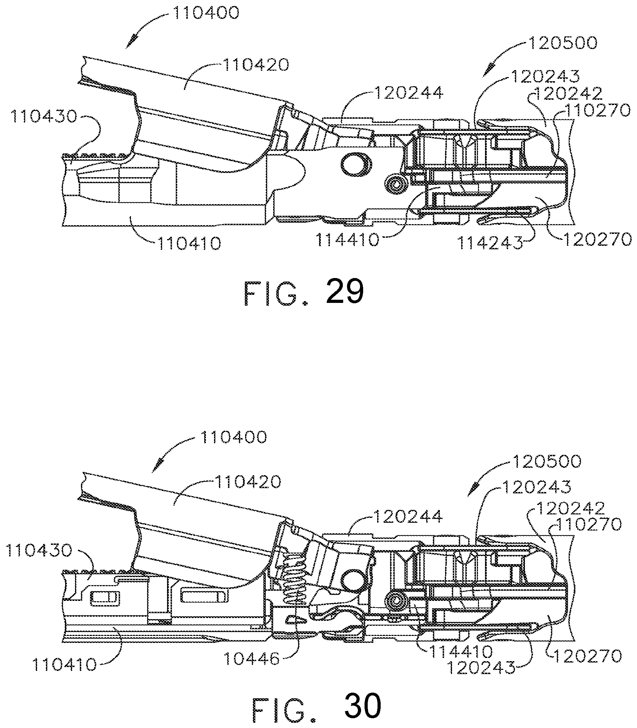

Once the firing rod 120160 and the firing bar 110710 have been retracted back into an unfired position, the cam 110722 of the tissue cutting knife 110720 is no longer engaged with the anvil jaw 110420. At such point, the anvil jaw 110420 can be re-opened. To re-open the anvil jaw 110420, the closure tube 120240 is retracted proximally to dis-engage the distal portion 120244 of the closure tube 120240 from the anvil jaw 110420 such that the springs 10446 (FIG. 30) in the end effector 110400 uncompress and rotate the anvil jaw 110420 into its unclamped position. Referring to FIGS. 23-25, the closure tube 120240 is retracted proximally when one or both of the release actuators 10180a and 10180b on the handle 120100 are actuated and the closure trigger 10140 is released. To this end, the handle 120100 comprises a spring that is compressed when the closure trigger 10140 is actuated and is configured to resiliently push the closure tube 120240 proximally when one or both of the release actuators 10180a and 10180b are actuated to unlock the closure drive. The retraction of the closure tube 120240 also disengages the closure tube 120240 from the clutch assembly. The clutch assembly includes a biasing member, such as a torsion spring, for example, which, in such instances, automatically rotates the clutch assembly and returns the stapling instrument from its staple firing mode into its articulation mode in which the articulation drive is re-engaged with the staple firing drive.

As discussed above, closing the end effector 110400 of the surgical instrument 120000 uncouples the articulation drive from the drive system 120900. Stated another way, the end effector 110400 cannot be articulated once it is closed. In various embodiments, further to the above, the closure drive of the surgical instrument 120000 comprises one or more partially-closed positions for the end effector 110400. In a partially-closed position of the end effector 110400, the anvil jaw 110420 is not in a completely-closed position. In such embodiments, and in such instances, the articulation drive is still operably coupled to the firing rod 120160 even though the anvil jaw 110420 is in a partially-closed position. As a result, a clinician can, in some instances, better visualize the positioning of a partially-closed end effector 110400 relative to the patient tissue while still being able to adjust the articulated position of the end effector 110400. In at least one embodiment, the handle 120100 comprises a detent, or latch, feature which allows the closure trigger to be moved into and held in a partially-closed position while the articulation mode of the surgical instrument 120000 is still active. Once the clinician is satisfied with the positioning of the end effector 110400, the clinician can completely close the closure trigger which, as a result, decouples the articulation drive from the drive system 120900 as discussed above. In various embodiments, such as those comprising an electric motor for driving the closure system, the handle 120100 can comprise an actuator, or button, in communication with the control system of the surgical instrument 120000 which, when actuated, causes the control system to move the anvil jaw 110420 into a partially-closed position in which the articulation mode is still active. Once the clinician is satisfied with the position of the end effector 110400, the clinician can depress another actuator, or button, in communication with the control system which, when actuated, causes the control system to move the anvil jaw 110420 into a completely-closed position and, as a result, decouples the articulation drive from the drive system 120900.

Further to the above, a staple cartridge is insertable into, and seatable within, a cartridge jaw of a stapling instrument. In various embodiments, the cartridge jaw comprises a stop, or datum, against which the staple cartridge can contact to make sure that the staple cartridge is seated in the correct position in the cartridge jaw. The stop is configured to prevent the staple cartridge from being inserted too deeply, i.e., proximally, into the end effector. Without such a stop, it is possible - in some instances and in some embodiments - for the staple cartridge to be inserted too deeply into the end effector such that the staple cartridge accidentally contacts the tissue cutting knife, for instance. In some such instances, the sled positioned in the staple cartridge may contact the tissue cutting knife before the staple cartridge is fully seated and, as a result, the sled may be accidentally, and prematurely, pushed distally within the staple cartridge from its unfired position. A sled in such a disturbed position would not be able to defeat or deactivate a staple firing lockout that keys off of the sled being in its unfired position at the outset of the staple firing stroke of the tissue cutting knife.

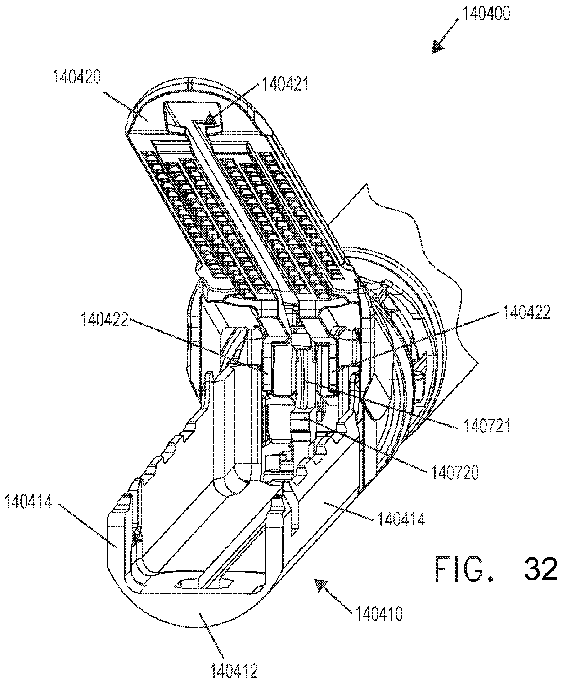

In addition to or in lieu of a cartridge stop in a cartridge jaw as discussed above, the anvil jaw of an end effector can comprise a cartridge stop configured to prevent a staple cartridge from being inserted too deeply, i.e., proximally, within the end effector. Referring to FIGS. 32-34, an end effector 140400 of a surgical stapling instrument comprises a cartridge jaw 140410 and an anvil jaw 140420. The anvil jaw 140420 is rotatable relative to the cartridge jaw 140410; however, in various other embodiments, the cartridge jaw 140410 is rotatable relative to the anvil jaw 140420. In either event, the cartridge jaw 140410 is configured to receive a staple cartridge 140430 therein. The cartridge jaw 140410 defines a channel including a bottom wall 140412 and lateral sidewalls 140414 extending upwardly from the bottom wall 140412. When the staple cartridge 140430 is inserted into the channel, a proximal end 140432 of the staple cartridge 140430 is inserted between the cartridge jaw 140410 and the anvil jaw 140420 by a clinician and moved proximally into the end effector 140400. In many instances, the clinician is able to visually align the staple cartridge 140430 between the lateral sidewalls 140414 and visually determine the proper depth, i.e., proximal-to-distal position, of the staple cartridge 140430 along a longitudinal axis LA before seating the staple cartridge 140430 into the cartridge jaw 140410. The cartridge jaw 140410 and/or the staple cartridge 140430 comprise snap-fit and/or press-fit features which releasably secure the staple cartridge 140430 in the cartridge jaw 140410 when the staple cartridge 140430 is pushed into, i.e., seated within, the cartridge jaw 140410. In some instances, however, the clinician may mistakenly attempt to insert the staple cartridge 140430 too deeply, i.e., proximally, into the end effector 140400 when attempting to seat the staple cartridge 140430 within the cartridge jaw 140410. As such, the anvil jaw 140420 comprises cartridge stops 140422 which are configured to prevent the staple cartridge 140430 from contacting a firing bar 140710 of the stapling instrument when the staple cartridge 140430 is inserted into the end effector 140400.

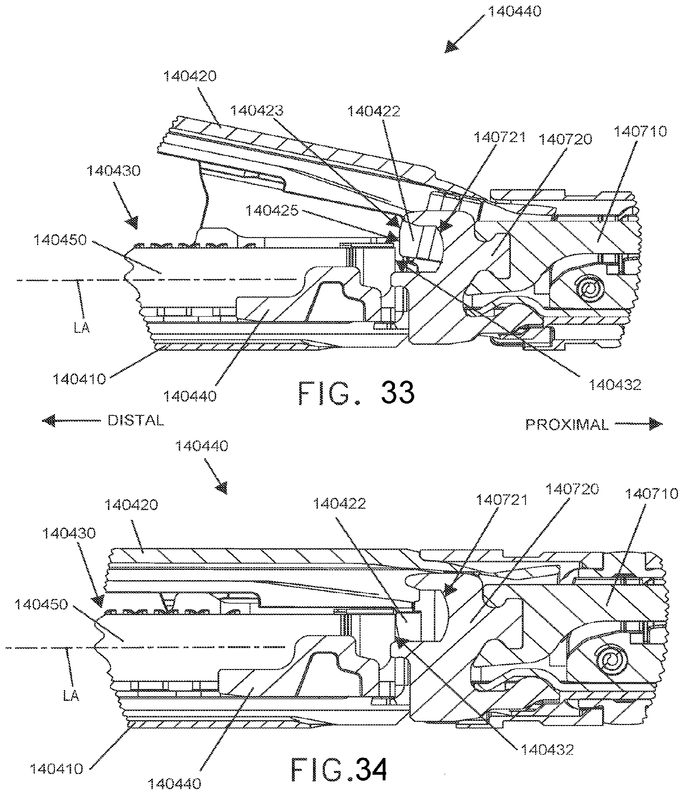

Further to the above, the firing bar 140710 comprises a distal knife head 140720 including a tissue knife edge 140721 configured to cut patient tissue during a staple firing stroke, a first cam configured to engage the anvil jaw 140420 during the staple firing stroke, and a second cam configured to engage the cartridge jaw 140410 during the staple firing stroke. When the anvil jaw 140420 is in its open configuration and the firing bar 140710 has not been advanced through the staple firing stroke, referring to FIG. 33, the cartridge stops 140422 extend distally with respect to the firing bar 140710 such that the staple cartridge 140430 cannot contact the firing bar 140710 when the staple cartridge 140430 is being loaded proximally into the end effector 140400. As a result, a sled 140440 positioned in a cartridge body 140450 of the staple cartridge 140430 isn't accidentally pushed forward out of its proximal unfired position (FIGS. 33 and 34) by accidentally contacting the firing bar 140710 prior to the staple firing stroke.

Once the staple cartridge 140430 has been properly seated in the cartridge jaw 140410, further to the above, the end effector 140400 can be moved into its clamped configuration (FIG. 34) such that the end effector 140400 can be inserted into a patient and/or clamped onto patient tissue. When the staple firing stroke is initiated, the firing bar 140710 is advanced distally past the cartridge stops 140422 to fire the staples stored in the cartridge body 140450. Stated another way, the cartridge stops 140422 are positioned and arranged such that they do not interfere with the firing bar 140710 during the staple firing stroke and/or when the firing bar 140710 is retracted back into its unfired position. Each cartridge stop 140422 comprises a tab, shoulder, wall, and/or flange, for example, extending downwardly from the anvil jaw 140420, i.e., toward the cartridge jaw 140410, such that the cartridge stops 140422 extend downwardly on the lateral sides of the firing bar 140710. That being said, the cartridge stops 140422 can comprise any suitable configuration and, in at least one embodiment, an anvil jaw 140420 may have only one cartridge stop 140422.

As a result of the above, the possibility of the sled 140440 being accidentally displaced distally out of its proximal unfired position prior to the staple firing stroke is reduced. When the sled 140440 is in its proximal unfired position when the staple firing stroke is initiated, further to the above, the sled 140440 can defeat or overcome a firing stroke lockout at the beginning of the staple firing stroke which permits the staple firing stroke to be performed. When the sled 140440 is not in its proximal unfired position when the staple firing stroke is initiated, the sled 140440 does not defeat or overcome the firing stroke lockout at the beginning of the staple firing stroke which prevents the staple firing stroke from being performed.

Further to the above, referring again to FIGS. 33 and 34, the proximal end 140432 of the staple cartridge 140430 is positioned close to, but distally with respect to, the cartridge stops 140422 when the staple cartridge 140430 is seated in the cartridge jaw 140410. The cartridge stops 140422 extend behind the proximal end 140432 of the staple cartridge 140430 when the end effector 140400 is in its open configuration (FIG. 33) and in its clamped configuration (FIG. 34) such that there is clearance between the cartridge 140430 and the cartridge stops 140422 so that the end effector 140400 can be opened and closed.

Notably, further to the above, the distal knife head 140720 moves within a longitudinal slot 140421 defined in the anvil jaw 140420 between the cartridge stops 140422 during the staple firing stroke. The cartridge stops 140422 extend behind the staple cartridge 140430 on opposite sides of the longitudinal slot 140421. Also notably, the distal knife head 140720 moves within a longitudinal slot defined in the staple cartridge 140430 during the staple firing stroke. The longitudinal slot defined in the staple cartridge 140430 divides the proximal end 140432 of the staple cartridge 140430 into a first lateral side and a second lateral side and is aligned with, or substantially aligned with, the longitudinal slot 140421 in the anvil jaw 140420. As a result of this arrangement, one of the cartridge stops 140422 extends behind the first lateral side of the staple cartridge 140430 and the other cartridge stop 140422 extends behind the second lateral side of the staple cartridge 140430.

Further to the above, each cartridge stop 140422 comprises a distal edge 140423 which faces the staple cartridge 140430. Each distal edge 140423 comprises one or more surfaces which are configured to block the proximal movement of the staple cartridge 140430 without contacting the sled 140440. For instance, referring to FIG. 33, the distal edge 140423 of each cartridge stop 140422 comprises a flat, or an at least substantially flat, surface 140425 which faces the staple cartridge 140430 when the end effector 140400 is in its open configuration and the staple cartridge 140430 is inserted into the end effector 140400. If the staple cartridge 140430 is inserted too deeply into the end effector 140400, the cartridge body 140450 of the staple cartridge 140430 contacts the cartridge stops 140422 instead of the sled 140440 contacting the cartridge stops 140422. If the sled 140440 were to contact the cartridge stops 140422, the staple cartridge 140430 would become spoiled and unuseable, as described above, and would need to be replaced. As a result of the arrangement of the end effector 140400, the staple cartridge 140430 may not be accidentally spoiled before it is fired which can reduce time during the surgical procedure and reduce the frustration of the clinician.

The surgical instrument systems described herein are motivated by an electric motor; however, the surgical instrument systems described herein can be motivated in any suitable manner. In certain instances, the motors disclosed herein may comprise a portion or portions of a robotically controlled system. U.S. Patent No. 9,072,535, for example, discloses several examples of a robotic surgical instrument system in greater detail. The disclosures of International Patent Publication No. WO 2017/083125, International Patent Publication No. WO 2017/083126, International Patent Publication No. WO 2015/153642, U.S. Patent Application Publication No. 2017/0265954, U.S. Patent Application Publication No. 2017/0265865, and U.S. Patent Publication No. 2017/0290586 are relevant.

The surgical instrument systems described herein have been described in connection with the deployment and deformation of staples; however, the embodiments described herein are not so limited. Various embodiments are envisioned which deploy fasteners other than staples, such as clamps or tacks, for example. Moreover, various embodiments are envisioned which utilize any suitable means for sealing tissue. For instance, an end effector in accordance with various embodiments can comprise electrodes configured to heat and seal the tissue. Also, for instance, an end effector in accordance with certain embodiments can apply vibrational energy to seal the tissue.

Although various devices have been described herein in connection with certain embodiments, modifications and variations to those embodiments may be implemented. Particular features, structures, or characteristics may be combined in any suitable manner in one or more embodiments. Thus, the particular features, structures, or characteristics illustrated or described in connection with one embodiment may be combined in whole or in part, with the features, structures or characteristics of one ore more other embodiments without limitation. Also, where materials are disclosed for certain components, other materials may be used. Furthermore, according to various embodiments, a single component may be replaced by multiple components, and multiple components may be replaced by a single component, to perform a given function or functions. The foregoing description and following claims are intended to cover all such modification and variations.

The devices disclosed herein can be designed to be disposed of after a single use, or they can be designed to be used multiple times. In either case, however, a device can be reconditioned for reuse after at least one use. Reconditioning can include any combination of the steps including, but not limited to, the disassembly of the device, followed by cleaning or replacement of particular pieces of the device, and subsequent reassembly of the device. In particular, a reconditioning facility and/or surgical team can disassemble a device and, after cleaning and/or replacing particular parts of the device, the device can be reassembled for subsequent use. Those skilled in the art will appreciate that reconditioning of a device can utilize a variety of techniques for disassembly, cleaning/replacement, and reassembly. Use of such techniques, and the resulting reconditioned device, are all within the scope of the present application.

The devices disclosed herein may be processed before surgery. First, a new or used instrument may be obtained and, when necessary, cleaned. The instrument may then be sterilized. In one sterilization technique, the instrument is placed in a closed and sealed container, such as a plastic or TYVEK bag. The container and instrument may then be placed in a field of radiation that can penetrate the container, such as gamma radiation, x-rays, and/or high-energy electrons. The radiation may kill bacteria on the instrument and in the container. The sterilized instrument may then be stored in the sterile container. The sealed container may keep the instrument sterile until it is opened in a medical facility. A device may also be sterilized using any other technique known in the art, including but not limited to beta radiation, gamma radiation, ethylene oxide, plasma peroxide, and/or steam.

This application is intended to cover any variations, uses, or adaptations of the invention using its general principles.

## Claims

1. A surgical end effector assembly (110400), comprising:
an anvil jaw (110420);
a cartridge channel jaw (110410);
a staple cartridge comprising a plurality of staples removably stored within said staple cartridge, wherein said staple cartridge is installable in said cartridge channel jaw (110410);
a firing member assembly actuatable through a firing stroke to deploy said staples from said staple cartridge, wherein said firing stroke comprises a beginning where said firing member assembly moves distally from a proximal-most position to an intermediate position, and wherein said firing member assembly comprises:
a firing bar (110710); and
a distal head portion (110720) connected to said firing bar (110710), wherein said distal head portion comprises:
a first jaw-camming member (110724) and a second jaw-camming member (110722) configured to control a tissue-gap distance between said anvil jaw (110420) and said cartridge channel jaw (110410) during said firing stroke; and
a tissue-cutting edge (110271); and
a lockout (10419) configured to stop said firing member assembly from moving distally beyond said lockout (10419); and
a lockout spring (110490), wherein:
the firing member assembly further comprises a tail extension (110729) extending proximally from said distal head portion (100720) below said firing bar (110710); wherein:
said lockout spring (110490) is configured to:
apply no force to said firing member assembly when said firing member assembly is in said proximal-most position; and
apply a downward locking force to said tail extension (110729) during the beginning of said firing stroke to bias said firing member assembly toward said lockout (10419).

2. The surgical end effector assembly of Claim 1, wherein said staple cartridge further comprises a sled movable between an unfired position and a fully-fired position by said firing member assembly, and wherein said sled is configured to prevent said firing member assembly from being blocked by said lockout (10491) when said sled is in said unfired position.

3. The surgical end effector assembly of Claim 2, wherein said distal head portion comprises a distal nose (110725), wherein said sled comprises a proximal ledge, and wherein said distal nose (110725) is configured to catch said proximal ledge when said sled is in said unfired position.

4. The surgical end effector assembly of Claim 1, wherein said lockout spring (110490) is configured to apply said downward locking force to said tail extension (110729) proximal to said tissue-cutting edge (110271).

5. The surgical end effector assembly of Claim 1, wherein said lockout spring (110490) comprises lateral support flanges (110495) positioned within recesses (110415) defined in said cartridge channel jaw (110410).

6. A surgical stapling assembly, comprising:
the surgical end effector assembly of claim 1, 4 or 5; and
a proximal shaft frame (110200); wherein
the proximal-most position is an unfired position;
the firing bar (110710) is a firing shaft;
the tissue-cutting edge (110271) comprises a knife; and
the lockout spring (110499) is attached to said proximal shaft frame (110200).

7. The surgical stapling assembly of Claim 6, wherein said staple cartridge further comprises a sled movable distally from an unspent position by said firing member assembly, and wherein said sled is configured to prevent said firing member assembly from being stopped by said lockout (10419) when said sled is in said unspent position.

8. The surgical stapling assembly of Claim 7, wherein said distal head portion (110720) comprises a distal nose (110725), wherein said sled comprises a proximal ledge, and wherein said distal nose (110725) is configured to catch said proximal ledge when said sled is in said unspent position.

9. A surgical stapling assembly, comprising:
the surgical end effector assembly of claim 1, 4 or 5;
wherein said staple cartridge comprises:
a cartridge body including a proximal end, a distal end, and rows of staple cavities extending between said proximal end and said distal end, wherein said staples are removably stored in said staple cavities; and
a sled positioned in an unfired position in said proximal end, wherein said sled is movable distally during said firing stroke to deploy said staples from said staple cavities;
wherein said firing member assembly is actuatable to move said sled through said firing stroke; wherein
said firing bar (11710) comprises a firing shaft;
said first jaw-camming member (110722) comprises an upper jaw-camming member configured to engage said anvil jaw (110420) during said firing stroke and said second jaw-camming member (110722) comprises a lower jaw-camming member configured to engage said cartridge channel jaw (110410) during said firing stroke; and
said lockout spring (110490) is in an initially-unloaded state prior to the beginning of said firing stroke, wherein said tail extension (110729) contacts said lockout spring (110490) during the beginning of said firing stroke such that said lockout spring (110490) applies said downward locking force to said tail extension (110729) to bias said distal head portion (110720) toward said lockout (10419), wherein said lockout (10419) is configured to block said firing member assembly from moving distally past said lockout (10419) when said staple cartridge is not seated in said cartridge channel jaw (110410), and wherein said sled is configured to support said distal head portion (110720) from being blocked by said lockout (10419) when said staple cartridge is seated in said cartridge channel jaw (110410) and said sled is in said unfired position.

10. The surgical stapling assembly of Claim 9, wherein said lockout (10419) comprises a lock shoulder (10417), and wherein said distal head portion (110720) comprises a lock surface (110725) extending laterally therefrom which is configured to contact said lock shoulder (10417) to block the distal advancement of said firing member assembly when said staple cartridge is not seated in said cartridge channel jaw (110410).

11. The surgical stapling assembly of Claim 10, wherein said tissue cutting edge (110271) does not extend distally beyond said lock shoulder (10417) when said lock surface (110725) contacts said lock shoulder (10417).

## Patentansprüche

1. Chirurgische Endeffektoranordnung (110400), umfassend:
eine Ambossbacke (110420);
eine Magazinkanalbacke (110410);
ein Klammermagazin, umfassend eine Vielzahl von Klammern, die entnehmbar innerhalb des Klammermagazins gelagert sind, wobei das Klammermagazin in der Magazinkanalbacke (110410) installierbar ist;
eine Auslöseelementanordnung, die durch einen Auslösehub betätigbar ist, um die Klammern aus dem Klammermagazin auszugeben, wobei der Auslösehub einen Anfang umfasst, an dem sich die Auslöseelementanordnung distal von einer proximalsten Position in eine Zwischenposition bewegt, und wobei die Auslöseelementanordnung umfasst:
einen Auslösestab (110710); und
einen distalen Kopfabschnitt (110720), der mit dem Auslösestab (110710) verbunden ist, wobei der distale Kopfabschnitt umfasst:
ein erstes Backennockenelement (110724) und ein zweites Backennockenelement (110722), die konfiguriert sind, um während des Auslösehubs einen Gewebespaltabstand zwischen der Ambossbacke (110420) und der Magazinkanalbacke (110410) zu steuern; und
eine Gewebeschneidkante (110271); und
eine Sperre (10419), die konfiguriert ist, um die Auslöseelementanordnung anzuhalten, sich distal über die Sperre (10419) hinaus zu bewegen; und
eine Sperrfeder (110490), wobei:
die Auslöseelementanordnung ferner eine hintere Endverlängerung (110729) umfasst, die sich proximal von dem distalen Kopfabschnitt (100720) unterhalb des Auslösestabs (110710) erstreckt; wobei:
die Sperrfeder (110490) konfigurier ist zum:
Ausüben keiner Kraft auf die Auslöseelementanordnung, wenn sich die Auslöseelementanordnung in der proximalsten Position befindet; und
Ausüben einer nach unten gerichteten Sperrkraft auf die hintere Endverlängerung (110729) während des Beginns des Auslösehubs, um die Auslöseelementanordnung in Richtung der Sperre (10419) vorzuspannen.

2. Chirurgische Endeffektoranordnung nach Anspruch 1, wobei das Klammermagazin ferner einen Schlitten umfasst, der durch die Auslöseelementanordnung zwischen einer nicht ausgelösten Position und einer vollständig ausgelösten Position bewegbar ist, und wobei der Schlitten konfiguriert ist, um zu verhindern, dass die Auslöseelementanordnung durch die Sperre (10491) blockiert wird, wenn sich der Schlitten in der nicht ausgelösten Position befindet.

3. Chirurgische Endeffektoranordnung nach Anspruch 2, wobei der distale Kopfabschnitt eine distale Nase (110725) umfasst, wobei der Schlitten eine proximale Leiste umfasst und wobei die distale Nase (110725) konfiguriert ist, um die proximale Leiste zu erfassen, wenn sich der Schlitten in der nicht ausgelösten Position befindet.

4. Chirurgische Endeffektoranordnung nach Anspruch 1, wobei die Sperrfeder (110490) konfiguriert ist, um die nach unten gerichtete Sperrkraft auf die hintere Envderlängerung (110729) proximal zu der Gewebeschneidkante (110271) auszuüben.

5. Chirurgische Endeffektoranordnung nach Anspruch 1, wobei die Sperrfeder (110490) seitliche Stützflansche (110495) umfasst, die innerhalb von Aussparungen (110415) positioniert sind, die in der Magazinkanalbacke (110410) definiert sind.

6. Chirurgische Klammeranordnung, umfassend:
die chirurgische Endeffektoranordnung nach Anspruch 1, 4 oder 5; und
einen proximalen Schaftrahmen (110200); wobei
die proximalste Position eine nicht ausgelöste Position ist;
der Auslösestab (110710) ein Auslöseschaft ist;
die Gewebeschneidkante (110271) ein Messer umfasst; und
die Sperrfeder (110499) an dem proximalen Schaftrahmen (110200) befestigt ist.

7. Chirurgische Klammeranordnung nach Anspruch 6, wobei das Klammermagazin ferner einen Schlitten umfasst, der durch die Auslöseelementanordnung distal aus einer nicht verbrauchten Position bewegbar ist, und wobei der Schlitten konfiguriert ist, um zu verhindern, dass die Auslöseelementanordnung durch die Sperre (10419) angehalten wird, wenn sich der Schlitten in der nicht verbrauchten Position befindet.

8. Chirurgische Klammeranordnung nach Anspruch 7, wobei der distale Kopfabschnitt (110720) eine distale Nase (110725) umfasst, wobei der Schlitten eine proximale Leiste umfasst und wobei die distale Nase (110725) konfiguriert ist, um die proximale Leiste zu erfassen, wenn sich der Schlitten in der nicht verbrauchten Position befindet.

9. Chirurgische Klammeranordnung, umfassend:
die chirurgische Endeffektoranordnung nach Anspruch 1, 4 oder 5;
wobei das Klammermagazin umfasst:
einen Magazinkörper, der ein proximales Ende, ein distales Ende und Reihen von Klammerhohlräumen einschließt, die sich zwischen dem proximalen Ende und dem distalen Ende erstrecken, wobei die Klammern entnehmbar in den Klammerhohlräumen gelagert werden; und
einen Schlitten, der in einer nicht ausgelösten Position in dem proximalen Ende positioniert ist, wobei der Schlitten während des Auslösehubs distal bewegbar ist, um die Klammern aus den Klammerhohlräumen auszugeben;
wobei die Auslöseelementanordnung betätigbar ist, um den Schlitten durch den Auslösehub zu bewegen; wobei
der Auslösestab (11710) einen Auslöseschaft umfasst;
das erste Backennockenelement (110722) ein oberes Backennockenelement umfasst, so konfiguriert ist, um während des Auslösehubs mit der Ambossbacke (110420) in Eingriff zu stehen, und das zweite Backennockenelement (110722) ein unteres Backennockenelement umfasst, das konfiguriert ist, um während des Auslösehubs mit der Magazinkanalbacke (110410) in Eingriff zu stehen; und
sich die Sperrfeder (110490) vor Beginn des Auslösehubs in einem zunächst ungeladenen Zustand befindet, wobei die hintere Endverlängerung (110729) während des Beginns des Auslösehubs die Sperrfeder (110490) derart berührt, dass die Sperrfeder (110490) die nach unten gerichtete Sperrkraft auf die hintere Endverlängerung (110729) ausübt, um den distalen Kopfabschnitt (110720) in Richtung der Sperre (10419) vorzuspannen, wobei die Sperre (10419) konfiguriert ist, um die Auslöseelementanordnung zu blockieren, sich distal über die Sperre (10419) hinaus zu bewegen, wenn das Klammermagazin nicht in der Magazinkanalbacke (110410) sitzt, und wobei der Schlitten konfiguriert ist, um den distalen Kopfabschnitt (110720) unterstützt, um nicht durch die Sperre (10419) blockiert zu werden, wenn das Klammermagazin in der Magazinkanalbacke (110410) sitzt und sich der Schlitten in der nicht ausgelösten Position befindet.

10. Chirurgische Klammeranordnung nach Anspruch 9, wobei die Sperre (10419) eine Sperrschulter (10417) umfasst und wobei der distale Kopfabschnitt (110720) eine Sperroberfläche (110725) umfasst, die sich seitlich davon erstreckt, die konfiguriert ist, um mit der Sperrschulter (10417) in Berührung zu stehen, um die distale Vorwärtsbewegung der Auslöseelementanordnung zu blockieren, wenn das Klammermagazin nicht in der Magazinkanalbacke (110410) sitzt.

11. Chirurgische Klammervorrichtung nach Anspruch 10, wobei sich die Gewebeschneidkante (110271) distal nicht über die Sperrschulter (10417) hinaus erstreckt, wenn die Sperroberfläche (110725) die Sperrschulter (10417) berührt.

## Revendications

1. Ensemble effecteur terminal chirurgical (110400), comprenant :
une mâchoire d'enclume (110420) ;
une mâchoire de canal de cartouche (110410) ;
une cartouche d'agrafes comprenant une pluralité d'agrafes stockées de manière amovible au sein de ladite cartouche d'agrafes, dans lequel ladite cartouche d'agrafes est installable dans ladite mâchoire de canal de cartouche (110410) ;
un ensemble élément de déclenchement actionnable par le biais d'une course de déclenchement pour déployer lesdites agrafes à partir de ladite cartouche d'agrafes, dans lequel ladite course de déclenchement comprend un début où ledit ensemble élément de déclenchement se déplace de manière distale d'une position la plus proximale à une position intermédiaire, et dans lequel ledit ensemble élément de déclenchement comprend :
une barre de déclenchement (110710) ; et
une partie de tête distale (110720) reliée à ladite barre de déclenchement (110710), dans lequel ladite partie de tête distale comprend :
un premier élément de came de mâchoire (110724) et un second élément de came de mâchoire (110722) conçus pour commander une distance d'écartement de tissu entre ladite mâchoire d'enclume (110420) et ladite mâchoire de canal de cartouche (110410) pendant ladite course de déclenchement ; et
un bord de coupe de tissu (110271) ; et
un verrouillage (10419) conçu pour arrêter le déplacement distal dudit ensemble élément de déclenchement au-delà dudit verrouillage (10419) ; et
un ressort de verrouillage (110490), dans lequel :
l'ensemble élément de déclenchement comprend en outre une extension de queue (110729) s'étendant proximalement à partir de ladite partie de tête distale (100720) en dessous de ladite barre de déclenchement (110710) ; dans lequel :
ledit ressort de verrouillage (110490) est conçu pour :
n'appliquer aucune force audit ensemble élément de déclenchement lorsque ledit ensemble élément de déclenchement est dans ladite position la plus proximale ; et
appliquer une force de verrouillage vers le bas à ladite extension de queue (110729) pendant le début de ladite course de déclenchement pour solliciter ledit ensemble élément de déclenchement en direction dudit verrouillage (10419).

2. Ensemble effecteur terminal chirurgical selon la revendication 1, dans lequel ladite cartouche d'agrafes comprend en outre un traîneau mobile entre une position non déclenchée et une position complètement déclenchée par ledit ensemble élément de déclenchement, et dans lequel ledit traîneau est conçu pour empêcher ledit ensemble élément de déclenchement d'être bloqué par ledit verrouillage (10491) lorsque ledit traîneau est dans ladite position non déclenchée.

3. Ensemble effecteur terminal chirurgical selon la revendication 2, dans lequel ladite partie de tête distale comprend un nez distal (110725), dans lequel ledit traîneau comprend un rebord proximal, et dans lequel ledit nez distal (110725) est conçu pour attraper ledit rebord proximal lorsque ledit traîneau est dans ladite position non déclenchée.

4. Ensemble effecteur terminal chirurgical selon la revendication 1, dans lequel ledit ressort de verrouillage (110490) est conçu pour appliquer ladite force de verrouillage vers le bas à ladite extension de queue (110729) de manière proximale par rapport audit bord de coupe de tissu (110271).

5. Ensemble effecteur terminal chirurgical selon la revendication 1, dans lequel ledit ressort de verrouillage (110490) comprend des brides de support latéral (110495) positionnées au sein d'évidements (110415) définis dans ladite mâchoire de canal de cartouche (110410).

6. Ensemble d'agrafage chirurgical, comprenant :
l'ensemble effecteur terminal chirurgical selon la revendication 1, 4 ou 5 ; et
un châssis de tige proximale (110200) ; dans lequel
la position la plus proximale est une position non déclenchée ;
la barre de déclenchement (110710) est une tige de déclenchement ;
le bord de coupe de tissu (110271) comprend un couteau ; et
le ressort de verrouillage (110499) est fixé audit châssis de tige proximale (110200).

7. Ensemble d'agrafage chirurgical selon la revendication 6, dans lequel ladite cartouche d'agrafes comprend en outre un traîneau mobile de manière distale par rapport à position inutilisée par ledit ensemble élément de déclenchement, et dans lequel ledit traîneau est conçu pour empêcher ledit ensemble élément de déclenchement d'être arrêté par ledit verrouillage (10419) lorsque ledit traîneau est dans ladite position inutilisée.

8. Ensemble d'agrafage chirurgical selon la revendication 7, dans lequel ladite partie de tête distale (110720) comprend un nez distal (110725), dans lequel ledit traîneau comprend un rebord proximal, et dans lequel ledit nez distal (110725) est conçu pour attraper ledit rebord proximal lorsque ledit traîneau est dans ladite position inutilisée.

9. Ensemble d'agrafage chirurgical, comprenant :
l'ensemble effecteur terminal chirurgical selon la revendication 1, 4 ou 5 ;
dans lequel ladite cartouche d'agrafes comprend :
un corps de cartouche comportant une extrémité proximale, une extrémité distale et des rangées de cavités d'agrafe s'étendant entre ladite extrémité proximale et ladite extrémité distale, dans lequel lesdites agrafes sont stockées de manière amovible dans lesdites cavités d'agrafe ; et
un traîneau positionné dans une position non déclenchée dans ladite extrémité proximale, dans lequel ledit traîneau est mobile distalement pendant ladite course de déclenchement pour déployer lesdites agrafes à partir desdites cavités d'agrafe ;
dans lequel ledit ensemble élément de déclenchement est actionnable pour déplacer ledit traîneau à travers ladite course de déclenchement ; dans lequel
ladite barre de déclenchement (11710) comprend une tige de déclenchement ;
ledit premier élément de came de mâchoire (110722) comprend un élément de came de mâchoire supérieure conçu pour venir en prise avec ladite mâchoire d'enclume (110420) pendant ladite course de déclenchement et ledit second élément de came de mâchoire (110722) comprend un élément de came de mâchoire inférieure conçu pour venir en prise avec ladite mâchoire de canal de cartouche (110410) pendant ladite course de déclenchement ; et
ledit ressort de verrouillage (110490) est dans un état initialement non chargé avant le début de ladite course de déclenchement, dans lequel ladite extension de queue (110729) vient en contact avec ledit ressort de verrouillage (110490) pendant le début de ladite course de déclenchement de telle sorte que ledit ressort de verrouillage (110490) applique ladite force de verrouillage vers le bas à ladite extension de queue (110729) pour solliciter ladite partie de tête distale (110720) en direction dudit verrouillage (10419), dans lequel ledit verrouillage (10419) est conçu pour bloquer un déplacement distal dudit ensemble élément de déclenchement au-delà dudit verrouillage (10419) lorsque ladite cartouche d'agrafes n'est pas installée dans ladite mâchoire de canal de cartouche (110410), et dans lequel ledit traîneau est conçu pour aider à ce que ladite partie de tête distale (110720) ne soit pas bloquée par ledit verrouillage (10419) lorsque ladite cartouche d'agrafes est installée dans ladite mâchoire de canal de cartouche (110410) et que ledit traîneau est dans ladite position non déclenchée.

10. Ensemble d'agrafage chirurgical selon la revendication 9, dans lequel ledit verrouillage (10419) comprend un épaulement de verrouillage (10417), et dans lequel ladite partie de tête distale (110720) comprend une surface de verrouillage (110725) s'étendant latéralement à partir de celle-ci qui est conçue pour venir en contact avec ledit épaulement de verrouillage (10417) pour bloquer l'avancement distal dudit ensemble élément de déclenchement lorsque ladite cartouche d'agrafes n'est pas installée dans ladite mâchoire de canal de cartouche (110410).

11. Ensemble d'agrafage chirurgical selon la revendication 10, dans lequel ledit bord de coupe de tissu (110271) ne s'étend pas distalement au-delà dudit épaulement de verrouillage (10417) lorsque ladite surface de verrouillage (110725) vient en contact avec ledit épaulement de verrouillage (10417).
